# EUROPEAN PATENT SPECIFICATION

(11) **EP 1 605 971 B1**
(45) Date of publication and mention of the grant of the patent: **26.05.2010**
(21) Application number: 04722764.0
(22) Date of filing: 23.03.2004
(51) Int. Cl.: A61K 39/205

(54) **IMMUNOGENIC COMPOSITION AND METHODS**
IMMUNOGENE ZUSAMMENSETZUNG UND VERFAHREN
COMPOSITIONS ET METHODES IMMUNOGENES

(30) Priority: 26.03.2003 US 457876 P; 23.02.2004 US 546733 P
(43) Date of publication of application: 21.12.2005
(73) Proprietor: Wyeth LLC, Madison, NJ 07940 (US)
(72) Inventor: ELDRIDGE, John, H., Somers, NY 10589 (US); ISRAEL, Zimra, R., New York, NY 10069 (US); EGAN, Michael, A., Chester, NY 10918 (US); UDEM, Stephen A., New York, NY 10023 (US)
(74) Representative: Denholm, Anna Marie
(86) International application number: PCT/US2004/006089
(87) International publication number: WO 2004/093906

(56) References cited:
- WO-A-96/34625
- WO-A-02/097091
- SUTTER G ET AL: "NOVEL VACCINE DELIVERY SYSTEMS: SOLUTIONS TO HIV VACCINE DILEMMAS?" AIDS, LONDON, GB, vol. 15, no. SUPPL 5, 2001, pages S139-S145, XP009014330 ISSN: 0269-9370
- ROSE NINA F ET AL: "An effective AIDS vaccine based on live attenuated vesicular stomatitis virus recombinants" CELL, vol. 106, no. 5, 7 September 2001 (2001-09-07), pages 539-549, XP002297996 ISSN: 0092-8674 cited in the application
- EGAN M A ET AL: "The use of cytokines and chemokines as genetic adjuvants for plasmid DNA vaccines" CLINICAL AND APPLIED IMMUNOLOGY REVIEWS 2002 UNITED STATES, vol. 2, no. 4-5, 2002, pages 255-287, XP002297997 ISSN: 1529-1049
- EO S K ET AL: "Prime-boost immunization with DNA vaccine: mucosal route of administration changes the rules." JOURNAL OF IMMUNOLOGY (BALTIMORE, MD. : 1950) 1 MAY 2001, vol. 166, no. 9, 1 May 2001 (2001-05-01), pages 5473-5479, XP002297998 ISSN: 0022-1767 cited in the application
- SHIVER J W ET AL: "Replication-incompetent adenoviral vaccine vector elicits effective anti-immunodeficiency-virus immunity" NATURE, MACMILLAN JOURNALS LTD. LONDON, GB, vol. 415, 17 February 2002 (2002-02-17), pages 331-335, XP002963543 ISSN: 0028-0836 cited in the application
- HAGLUND K ET AL: "High-level primary CD8+ T-cell response to human immunodeficiency virus type 1 Gag and Env generated by vaccination with recombinant vesicular stomatitis viruses" JOURNAL OF VIROLOGY, THE AMERICAN SOCIETY FOR MICROBIOLOGY, US, vol. 76, no. 6, March 2002 (2002-03), pages 2730-2738, XP002266674 ISSN: 0022-538X cited in the application
- ROBERTS A ET AL: "Attenuated vesicular stomatitis viruses as vaccine vectors" JOURNAL OF VIROLOGY, THE AMERICAN SOCIETY FOR MICROBIOLOGY, US, vol. 73, no. 5, May 1999 (1999-05), pages 3723-3732, XP002266675 ISSN: 0022-538X cited in the application

### SEQUENCE LISTING

<110> Wyeth
   Eldridge, John H.
   Israel, Zimra R.
   Egan, Michael A.
   Udem, Stephen A.
<120> Immunogenic Composition and Methods
<130> AM-101319PCT
<150> US 60/457,876
   <151> 2003-03-26
<150> US 60/546,733
   <151> 2004-02-23
<160> 11
<170> PatentIn version 3.2
<210> 1
   <211> 42
   <212> PRT
   <213> Artificial
<220>
   <223> beta-amyloid 1-42 peptide
<400> 1
<210> 2
   <211> 31
   <212> DNA
   <213> Artificial
<220>
   <223> complementary primer
<400> 2
   cccctagtgg gatctccagt gatatttgga c 31
<210> 3
   <211> 31
   <212> DNA
   <213> Artificial
<220>
   <223> complementary primer
<400> 3
   gtccaaatat cactggagat cccactaggg g 31
<210> 4
   <211> 6
   <212> DNA
   <213> Artificial
<220>
   <223> complementary primer
<400> 4
   ctcgag 6
<210> 5
   <211> 6
   <212> DNA
   <213> Artificial
<220>
   <223> complementary primer
<400> 5
   ctccag 6
<210> 6
   <211> 36
   <212> DNA
   <213> Artificial
<220>
   <223> forward primer
<400> 6
   gatcgatcga attcacgcgt aacatgactt cttcag 36
<210> 7
   <211> 70
   <212> DNA
   <213> Artificial
<220>
   <223> reverse primer
<400> 7
   gaacggtcga cgcgcctcga gcgtgatatc tgttagtttt tttcatatca tgttgttggg 60
   cttgaagatc 70
<210> 8
   <211> 46
   <212> DNA
   <213> Artificial
<220>
   <223> forward primer
<400> 8
   gatcgatcga attcacgcgt aatatgttgt cttatctaat ctttgc 46
<210> 9
   <211> 73
   <212> DNA
   <213> Artificial
<220>
   <223> reverse primer
<400> 9
   ggaacggtcg acgcgcctcg agcgtgatat ctgttagttt ttttcatatt aacggaaatg 60
   agccatttcc acg 73
<210> 10
   <211> 4
   <212> PRT
   <213> HIV Env 89.6 G gene
<400> 10
   Asn Arg Val Arg
   1
<210> 11
   <211> 4
   <212> PRT
   <213> VSV G cytoplasmic tail
<400> 11
   Ile His Leu Cys
   1

## Description

### BACKGROUND OF THE INVENTION

To enhance the efficacy of immunogenic compositions, a variety of immunogenic compositions and methods have been reported using protein compositions, plasmid-based compositions, and recombinant virus constructs as immunogenic compositions. Prior studies have demonstrated that plasmid-based immunogenic compositions, upon systemic application, prime the systemic immune system to a second systemic immunization with a traditional antigen, such as a protein or a recombinant virus (See, e.g., Xiang et al., 1997 Springer Semin. Immunopathol., 19:257-268; Schneider, J et al,1998 Nature Med, 4:397; and Sedeguh, M. et al., 1998 Proc. Natl. Acad. Sci., USA 95:7648; Rogers, W. O. et al, 2001 Infec. & Immun., 69(9):5565-72; Eo, S. K, et al, 2001 J. Immunol., 166(9):5473-9; Ramshaw L A. and Ramsay, A. J., 2000 Immunol. Today, 21(4):163-5). For a review of known vaccine delivery systems with respect to HIV, see Sutter et al., 2001 AIDS 15(supplement 5): S139-S145.

An often used DNA prime/live vector boost regimen involves vaccinia viruses for the boost. Examples in the recent literature include such immunization for human immunodeficiency virus (HIV) (Hanke T. et al, 2002 Vaccine. 20(15):1995-8; Amara R.R. et al, 2002 Vaccine. 20(15):1949-55; Wee B.G. et al, 2002 J. Gen. Virol., 83(Pt1):75-80; Amara R.R. et al, 2001 Science. 292(5514):69-74). Prime-boost immunization with DNA and modified vaccinia virus vectors expressing antigens such as herpes simplex virus-2 glycoprotein D, *Leishmania infantum* P36/LACK antigen, *Plasmodium falciparum* TRAP antigen, HIV/SIV antigens, murine tuberculosis antigens, and influenza antigens, have been reported to elicit specific antibody and cytokine responses (See, e.g., Meseda C.A. et al., 2002 J. Infect. Dis., 186(8):1065-73; Amara RR. et al, 2002 J. Virol, 76(15):7625-31; Gonzalo RM. et al, 2002 Vaccine. 20(7-8):1226-31; Schneider J. et al, 2001 Vaccine, 19(32):4595-602; Hel Z. et al, 2001 J. Immunol, 167(12):7180-91; McShame H. et al, 2001 Infec. & Immun, 69(2):681-6 and Dagano P. et al 1999 Vaccine, 18(7-8):623-32 influenza and malaria models).

Plasmid prime-adenovirus boost genetic immunization regimens have recently been reported to induce alpha-fetoprotein-specific tumor immunity and to protect swine from classical swine fever (See, e.g., Meng W.S. 2001 Cancer Res., 61(24):8782-6; Hammond, J.M. et al, 2001 Vet. Microbio, 80(2):101-19; and United States Patent No. 6,210,663).

Other DNA plasmid prime-virus boost regimens have been reported. See, e.g, Matano T. et al, 2001 J. Virol., 75(23):11891-6 (a DNA prime/Sendai virus vector boost). DNA priming with recombinant poxvirus boosting has been reported for HIV-1 treatment (See, e.g., Kent, S.J. et al, 1998 J. Virol, 72:10180-8; Robinson, H.L. et al, 1999 Nat. Med, 5:526-34; and Tartaglia, J. et al, 1998 AIDS Res. Human Retrovirus., 14:S291-8).

Recombinant VSV vectors have been described in Haglund et al., 2002 Journal of Virology 76(6):2730-2738, WO 96/34625, and WO 02/097091. While a number of DNA prime/viral boost regimens are being evaluated, currently described immunization regimens have several disadvantages. For example, some of these above-noted viruses cause disease symptoms in subjects; others may result in recombination *in vivo*. Still other viruses are difficult to manufacture and/or have a limited ability to accept foreign genes. Still other viruses have disadvantages caused by significant pro-existing vector immunity in man, and other safety concerns.

There remains a need in the art for novel and useful immunization regimens that can produce enhanced levels of and humoral immune responses to the antigens in question and meet the requirements of safety, case of manufactured and the ability to overcome the mammalian hosts natural immune response to the vectors upon booster immunization.

### SUMMARY OF THE INVENTION

The present invention relates to a novel method and composition, and provides a kit for inducing in a mammalian subject an immune response against a pathogenic antigen or other antigen via a prime/boost regimen that shows a surprising synergistic stimulation of cellular immune response to the antigen compared to results obtained with either the DNA plasmid component or the recombinant viral component, when administered individually.

In one embodiment, the invention relates to a method of inducing an antigen-specific immune response in a mammalian subject. The method involves administering to the subject an effective amount of a first composition comprising a DNA plasmid comprising a DNA sequence encoding an antigen under the control of regulatory sequences directing expression thereof in a mammalian cell by the DNA plasmid. The method further involves administering to the subject an effective amount of a second composition comprising a recombinant vesicular stomatitis virus (rVSV) comprising a nucleic acid sequence encoding the antigen under the control of regulatory sequences directing expression thereof in the mammalian cell by the rVSV. In one embodiment, the recombinant VSV is an attenuated, replication competent virus. In another embodiment, the recombinant VSV is a non-replicating virus. The administrations of the first and second compositions may be in any order. Further, the invention contemplates multiple administrations of one of the compositions followed by multiple administrations of the other composition. In one embodiment, a cytokine is preferably co-administered.

In another embodiment the invention relates to an immunogenic composition for inducing an antigen-specific immune response to an antigen in a mammalian subject The immunogenic composition comprises a fast composition comprising a DNA plasmid comprising a DNA sequence encoding the antigen under the control of regulatory sequences directing expression thereof by the DNA plasmid. This composition also includes at least one replication competent, recombinant vesicular stomatitis virus (VSV) comprising a nucleic acid sequence encoding the same antigen under the control of regulatory sequences directing expression thereof by the recombinant VSV.

In yet another embodiment, the invention provides a kit for use in a therapeutic or prophylactic method of inducing an increased level of antigen-specific immune response in a mammalian subject. The kit includes, *inter alia,* at least one first composition comprising a DNA plasmid comprising a DNA sequence encoding an antigen under the control of regulatory sequences directing expression thereof in a mammalian cell; at least one second composition comprising a replication competent, recombinant vesicular stomatitis virus (rVSV) comprising a nucleic acid sequence encoding said antigen under the control of regulatory sequences directing expression thereof in said mammalian cell; and instructions for practicing the above-recited method.

In another embodiment, the invention provides the use of the above-described immunogenic composition or components thereof in the preparation of a medicament for inducing an immune response in an animal to the antigen employed in the composition.

Other aspects and embodiment of the present invention are disclosed in the following detailed description.

### BRIEF DESCRIPTION OF THE DRAWINGS

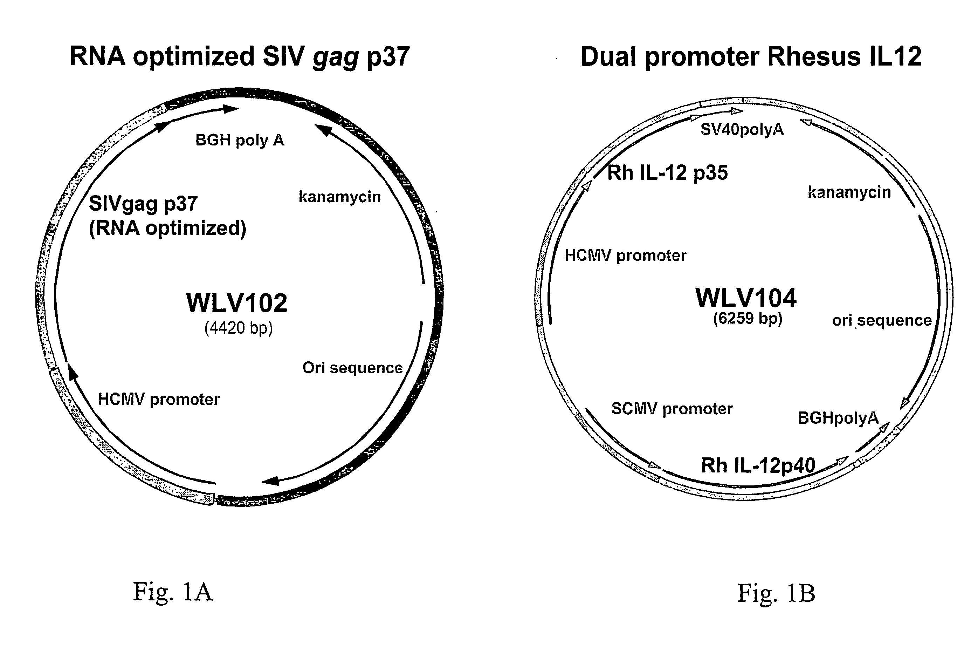

FIG. 1A is a schematic diagram of an illustrative plasmid DNA encoding a simian immunodeficiency virus (SIV) gag p37 protein. The diagram shows that the plasmid contains a human cytomegalovirus (HCMV) promoter/enhancer driving expression of the gag protein, a bovine growth hormone polyadenylation site (BGH polyA), an origin of replication sequence (ori) and a kanamycin resistance (*kan^{R}* ) marker gene.
FIG. 1B is a schematic diagram of an illustrative bicistronic plasmid DNA encoding the two subunits p35 and p40 of rhesus interleukin 12. The p35 subunit is under the control of the HCMV promoter and has an SV40 poly A site. The p40 subunit is under the control of the simian cytomegalovirus (SCMV) promoter and has a BGH poly A site, and is transcribed in the reverse direction. This plasmid also contains an ori sequence and a *kan^{R}* gene.

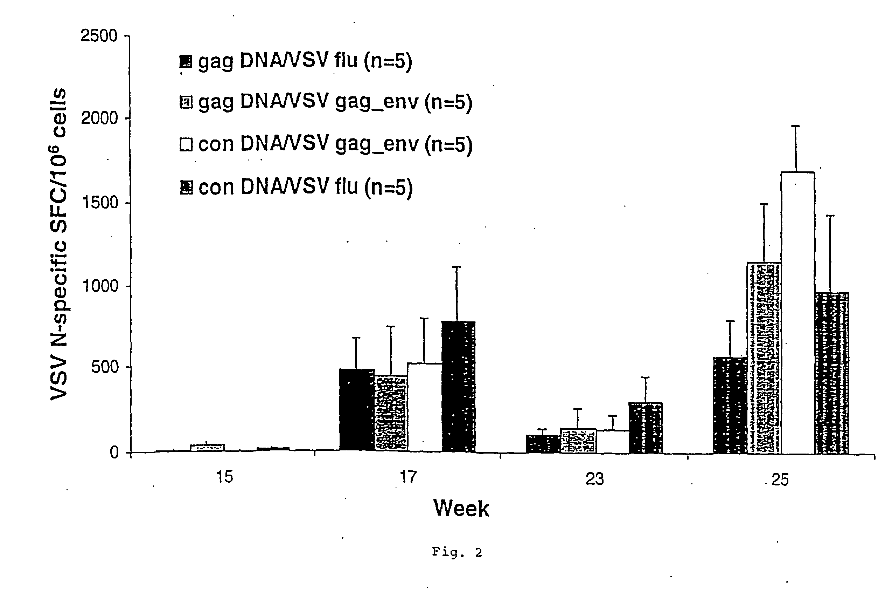

FIG. 2 is a bar graph showing VSV N-specific gamma interferon (IFN-γ) ELISpot responses in unfractionated peripheral blood mononuclear cells (PBMC) from animals immunized by a prime/boost regimen of the present invention. The leftmost dark bars represent a protocol of immunization with the DNA plasmid encoding the SIV gag protein with a boost of a VSV vector expressing an influenza hemagglutinin (flu HA) protein. Light gray bars represent a protocol of the invention involving a priming DNA gag plasmid immunization followed by a VSV boost expressing the HIV gag and env proteins. The pale bars represent a protocol involving a priming immunization with an empty or control DNA plasmid (con DNA) followed by immunization with a VSV expressing the HIV gag and env proteins. The rightmost dark bars represent a protocol involving a priming immunization with con DNA plasmid followed by immunization with a VSV expressing flu HA protein. Each group represents results from 5 animals.

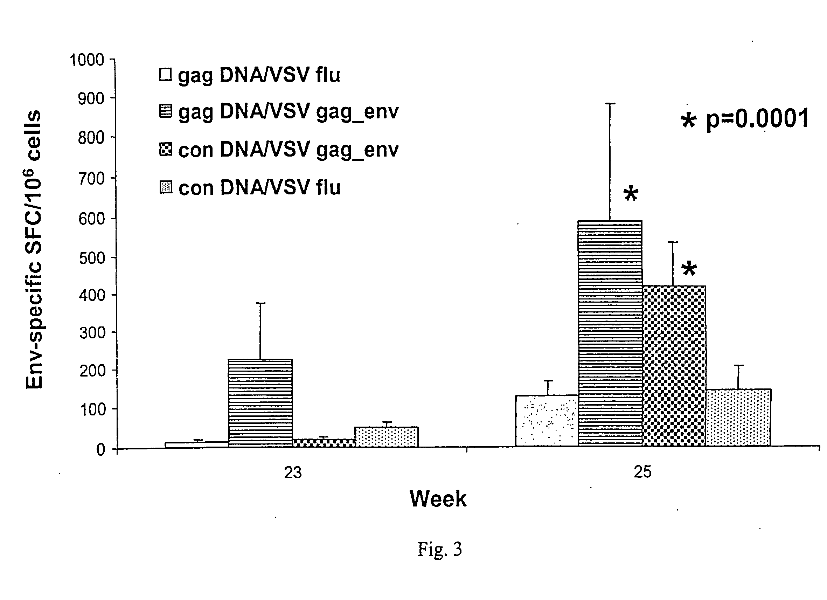

FIG. 3 is a bar graph showing HIVenv 6101-specific gamma interferon (IFN-γ) ELISpot responses in unfractionated PBMC from animals immunized by a prime/boost regimen of the present invention. The leftmost light gray bars represent a protocol of immunization with the DNA plasmid encoding the SIV gag protein with a boost of a VSV vector expressing flu HA protein. The striped bars represent a protocol of the invention involving a priming DNA gag plasmid immunization followed by a VSV boost expressing the HIV gag and env proteins. The checkerboard bars represent a protocol involving a priming immunization with an empty control DNA followed by immunization with a VSV expressing the HIV gag and env proteins. The dotted bars represent a protocol involving a priming immunization with control DNA plasmid followed by immunization with a VSV boost expressing flu HA protein. Each group represents results from 5 animals. The asterisks indicate where statistically significant differences occurred at p=0.0001.

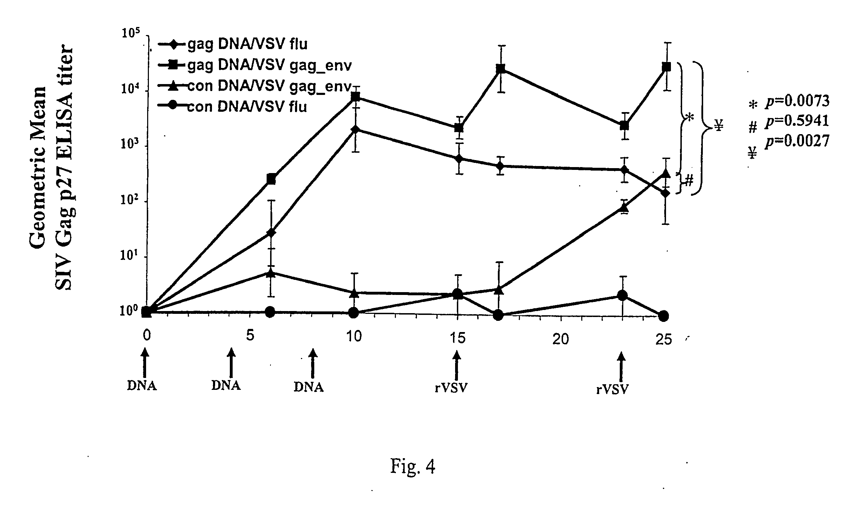

FIG. 4 is a graph showing serum anti-SIV gag p27 antibody titer by enzyme-linked immunosorbent assay (ELISA) for animals immunized by a prime/boost regimen of the present invention. Plasmid DNA was administered on day 0, week 4 and week 8 and VSV (serotype Indiana G) and VSV (serotype Chandipura G) boosts were administered on week 15 and 23, respectively. A protocol of immunization with the DNA plasmid encoding the SIV gag protein with a boost of a VSV vector expressing flu HA protein is represented by (◆). A protocol of the invention involving a priming DNA gag plasmid immunization followed by a VSV boost expressing the HIV gag and env proteins is represented by (■). A protocol involving a priming immunization with an empty control DNA followed by immunization with a VSV expressing the HIV gag and env proteins is represented by (▲). A protocol involving a priming immunization with control DNA plasmid followed by immunization with a VSV expressing flu HA protein is represented by (●). Each group represents results from 5 animals. Statistically significant differences between groups are shown as p=0.0073 (*); p=0-5941 (#) or p=0.0027 (¥).

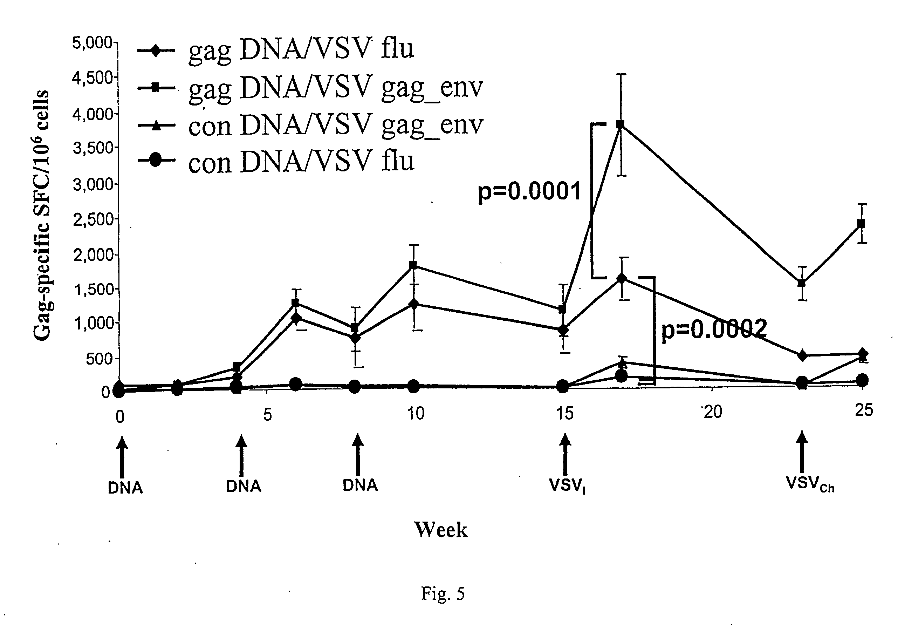

FIG. 5 is a graph showing SIV gag-specific spot forming cells per million cells evaluated by ELISpot assay for animals immunized by a prime/boost regimen of the present invention. Plasmid DNA was administered on day 0, week 4 and week 8 and VSV (serotype Indiana G) and VSV (serotype Chandipura G) boosts were administered on week 15 and 23, respectively. A protocol of immunization with the DNA plasmid encoding the SIV gag protein with a boost of a VSV vector expressing flu HA protein is represented by (◆). A protocol of the invention involving a priming DNA gag plasmid immunization followed by a VSV boost expressing the HIV gag and env proteins is represented by (■). A protocol involving a priming immunization with an empty control DNA followed by immunization with a VSV expressing the HIV gag and env proteins is represented by (▲). The (●) represent a protocol involving a priming immunization with control DNA plasmid followed by immunization with a VSV expressing flu HA protein. Each group represents results from 5 animals. Statistically significant differences between groups are indicated by brackets for p=0.0001 and p=0.0002.

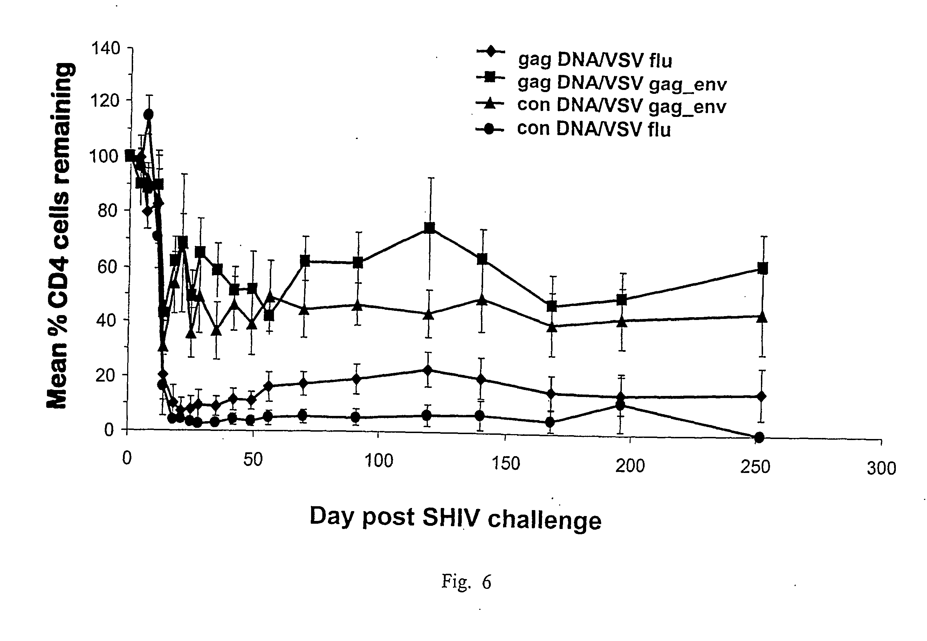

FIG. 6 is a graph showing the elevated immune responses elicited by the prime/boost combinations indicated by the same symbols as in Fig.5 results in increased protection from AIDS, as measured by a decreased loss of CD4 T-cells cells in days after challenge

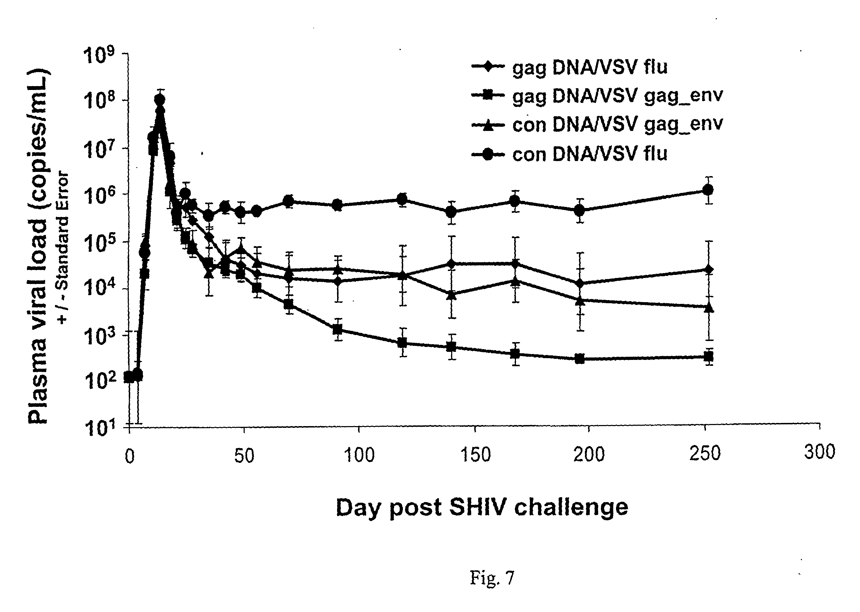

FIG. 7 is a graph showing the elevated immune responses elicited by the prime/boost combinations indicated by the same symbols as in Fig. 5 results in increased protection from AIDS, as measured by a decrease in circulating virus in plasma (virus copies/ml) in days after challenge.

### DETAILED DESCRIPTION OF THE INVENTION

The invention relates to a method of inducing an antigen-specific immune response in a mammalian subject or vertebrate subject by using in combination certain components of immunogenic compositions described in the prior art, and optimizing the components to produce surprising and synergistic results. Generally, the method involves administering to the subject an effective amount of a composition that includes a DNA plasmid comprising a DNA sequence encoding an antigen under the control of regulatory sequences directing expression thereof in a mammalian or vertebrate cell by the DNA plasmid. The method also includes a step of administering to the subject an effective amount of a composition comprising a replication competent, recombinant vesicular stomatitis virus (rVSV). This rVSV comprises a nucleic acid sequence encoding the antigen under the control of regulatory sequences directing expression thereof in the mammalian or vertebrate cell by the rVSV.

The first of these two immunogenic compositions to be administered in order is referred to as the priming composition. The second of these two immunogenic compositions to be administered in order is referred to as the boosting composition. Thus, either the DNA composition may be administered as the priming composition and the VSV vector composition administered as the boosting composition or vice versa. In one embodiment, the priming composition is administered to the subject at least once or multiple times prior to administration of the boosting composition. Thereafter, the boosting composition is subsequently administered to the subject at least once or multiple times. Further the invention contemplates multiple administrations of one of the compositions followed by multiple administrations of the other composition. The method further contemplates administering an effective amount of a cytokine as a step in the method.

It has been surprisingly found that practice of this method induces in a mammalian subject an immune response including an increase in CD8+ T cell response to the antigen greater than that achieved by administering the DNA plasmid or recombinant VSV alone. In fact, as indicated in the examples below, the immune response is greater than that expected by an additive combination of the two immunogenic compositions. Thus, the immune response induced by the novel method is a dramatic and synergistic increase in cellular and/or antibody responses to the antigen. See, e.g., Table 1 below and FIGs. 4 and 5, in which the peak antigen specific cellular response to the HIV-1 gag is over 3-fold higher than the response to administration of a DNA plasmid immunogenic composition alone or almost 9-fold higher than the response to the administration of the rVSV immunogenic composition alone.

While it is contemplated that the mammalian subject is a primate, preferably a human, the invention is not limited by the identification of the mammalian subject The components of this method are described in detail below and with reference to the cited documents to provide detail known to one of skill in the art.

### A. DNA. Plasmid Immunogenic Composition

Immunogenic compositions of this invention include a DNA plasmid comprising a DNA sequence encoding a selected antigen to which an immune response is desired. In the plasmid, the selected antigen is under the control of regulatory sequences directing expression thereof in a mammalian or vertebrate cell. The components of the plasmid itself are conventional.

Non-viral, plasmid vectors useful in this invention contain isolated and purified DNA sequences comprising DNA sequences that encode the selected immunogenic antigen. The DNA molecule may be derived from viral or non-viral, e.g., bacterial species that have been designed to encode an exogenous or heterologous nucleic acid sequence. Such plasmids or vectors can include sequences from viruses or phages. A variety of non-viral vectors are known in the art and may include, without limitation, plasmids, bacterial vectors, bacteriophage vectors, "naked" DNA and DNA condensed with cationic lipids or polymers.

Examples of bacterial vectors include, but are not limited to, sequences derived from *bacille Calmette Guérin* (BCG), *Salmonella, Shigella, E. coli,* and *Listeria*, among others. Suitable plasmid vectors include, for example, pBR322, pBR325, pACYC177, pACYC184, pUC8, pUC9, pUC18, pUC19, pLG339. pR290, pK37, pKC101,pAC105, pVA51, pKH47, pUB110, pMB9, pBR325, Col E1, pSC101, pBR313, pML21, RSF2124, pCR1, RP4, pBAD18, and pBR328.

Examples of suitable inducible *Escherichia coli* expression vectors include pTrc (Amann et al,1988 Gene, 69.301-315), the arabinose expression vectors (e.g., pBAD18, Guzman et al, 1995 J. Bacteriol., 177:4121-4130), and pETIId (Studier et al.,1990 Methods in Enzymology, 185:60-89). Target gene expression from the pTrc vector relies on host RNA polymerase transcription from a hybrid tip-lac fusion promoter. Target gene expression from the pETIId vector relies on transcription from a 77 gn10-lac fusion promoter mediated by a coexpressed viral RNA polymerase T7 gn1. This viral polymerase is supplied by host strains BL21 (DE3) or HMS I74(D133) from a resident prophage harboring a a *T7 gn1* gene under the transcriptional control of the *lacUV5* promoter. The pBAD system relies on the inducible arabinose promoter that is regulated by the *ara*C gene. The promoter is induced in the presence of arabinose.

The promoter and other regulatory sequences that drive expression of the antigen in the desired mammalian or vertebrate host may similarly be selected from a wide list of promoters known to be useful for that purpose. A variety of such promoters are disclosed below. In an embodiment of the immunogenic DNA plasmid composition described below, useful promoters are the human cytomegalovirus (HCMV) promoter/enhancer (described in, e.g., US Patent Nos. 5,168,062 and 5,385,839) and the SCMV promoter enhancer.

Additional regulatory sequences for inclusion in a nucleic acid sequence, molecule or vector of this invention include, without limitation, an enhancer sequence, a polyadenylation sequence, a splice donor sequence and a splice acceptor sequence, a site for transcription initiation and termination positioned at the beginning and end, respectively, of the polypeptide to be translated, a ribosome binding site for translation in the transcribed region, an epitope tag, a nuclear localization sequence, an IRES element, a Goldberg-Hogness "TATA" element, a restriction enzyme cleavage site, a selectable marker and the like. Enhancer sequences include, e.g., the 72 bp tandem repeat of SV40 DNA or the retroviral long terminal repeats or LTRs, etc. and are employed to increase transcriptional efficiency.

These other components useful in DNA plasmids, including, e.g., origins of replication, polyadenylation sequences (e.g., BGH polyA, SV40 polyA), drug resistance markers (e.g., kanamycin resistance), and the like may also be selected from among widely known sequences, including those described in the examples and mentioned specifically below.

Selection of promoters and other common vector elements are conventional and many such sequences are available with which to design the plasmids useful in this invention. See, e.g., Sambrook et al, Molecular Cloning. A Laboratory Manual, Cold Spring Harbor Laboratory, New York, (1989) and references cited therein at, for example, pages 3.18-326 and 16.17-1627 and Ausubel et al., Current Protocols in Molecular Biology, John Wiley & Sons, New York (1989). All components of the plasmids useful in this invention may be readily selected by one of skill in the art from among known materials in the art and available from the industry. Selection of plasmid components and regulatory sequences are not considered a limitation on this invention.

Examples of suitable DNA plasmid constructs for use in immunogenic compositions are described in detail in the following patent publications, e.g., International Patent Publication Nos. WO98/17799, WO99/43839 and WO98/17799; and United States Patent Nos. 5,593,972; 5,817,637; 5,830,876; and 5,891,505, among others.

Similarly, the selected antigen may be an antigen identified in the discussion below. In one embodiment of the immunogenic compositions herein, the selected antigen is an HIV-1 antigen, such as one expressed by *gag pol, env, nef, vpr, vpu, vif* and *tat.* Preferably the antigen sequence and other components of the DNA plasmid are optimized, such as by codon selection appropriate to the intended host and by removal of any inhibitory sequences, also discussed below with regard to antigen preparation.

This immunogenic composition may include therefore one plasmid encoding a single selected antigen for expression in the host. According to the present method, the plasmid composition also comprises one DNA plasmid comprising a DNA sequence encoding more than one copy of the same selected antigen. Alternatively, the composition may contain one plasmid expressing multiple selected antigens. Each antigen may be under the control of separate regulatory elements or components. Alternatively, each antigen may be under the control of the same regulators elements. In still another embodiment, the DNA plasmid composition may contain multiple plasmids, wherein each DNA plasmid encodes the same or a different antigen.

In still a further embodiment, the DNA plasmid immunogenic composition may further contain, as an individual DNA plasmid component or as part of the antigen-containing DNA plasmid, a nucleotide sequence that encodes a desirable cytokine, lymphokine or other genetic adjuvant. A host of such suitable adjuvants for which nucleic acid sequences are available are identified below. In the embodiments exemplified in this invention, a desirable cytokine for administration with the DNA plasmid composition of this invention is Interleukin-12.

The DNA plasmid composition is desirably administered in a pharmaceutically acceptable diluent, excipient or carrier, such as those discussed below. Although the composition may be administered by any selected route of administration, in one embodiment a desirable method of administration is coadministration intramuscularly of a composition comprising the plasmids with bupivacaine as the facilitating agent, described below.

In one embodiment of the method of this invention, in which the DNA composition is the priming composition, the method includes administering at least one DNA plasmid prior to the rVSV, the plasmid comprising a sequence encoding an antigen to which an immune response is desired to be induced. In another embodiment, the DNA priming composition further consists of a second plasmid encoding a selected cytokine. In still another embodiment, exemplified below, the DNA priming composition includes three plasmids, one plasmid expressing a first antigen, the second plasmid expressing the second different antigen and a third plasmid expressing a selected cytokine adjuvant. As detailed in the examples, one embodiment of a DNA priming composition contains three optimized plasmids, (1) a plasmid encoding an RNA optimized SIV *gag p37* gene (see FIG. 1A); (2) a plasmid encoding the two rhesus IL-12 subunits p35 and p40, under individual control of two promoters (see FIG. 1B) and (3) a plasmid encoding the HIV-1 gp160 env gene.

When used as a priming composition, this DNA plasmid composition is administered once or preferably, more than once prior to the boosting rVSV composition. When used as the boosting composition, this DNA plasmid composition is administered once or preferably, more than once after administration of the priming rVSV composition.

### B. rVSV Immunogenic Composition

Another immunogenic composition useful in the methods and compositions of this invention is a replication competent, live, attenuated, vesicular stomatitis virus (VSV) delivery vehicle. This recombinant VSV comprises a nucleic acid sequence encoding the selected antigen under the control of regulatory sequences directing expression thereof in the mammalian or vertebrate cell. Recombinant VSV vectors have been described in Haglund et al., 2002 Journal of Virology 76(6):2730-2738, WO 96/34625, and WO 02/097091. In the methods of this invention, the antigen used in the DNA plasmid composition is the same antigen used in the rVSV immunogenic composition.

VSV is a cattle virus, which is a member of the taxonomic Order designated Mononegavirales, and comprises an 11 kb nonsegmented, negative-strand RNA genome that encodes four internal structural proteins and one exterior transmembrane protein. In 3' to 5' order, the genes encode proteins designated the nucleocapsid (N), phosphoprotein (P), matrix protein (M), transmembrane glycoprotein (G) and polymerase (L). A live VSV may be isolated and "rescued" using techniques known in the art. See, e.g, US Patent Nos. 6,044,886; 6,168,943; and 5,789,299; International Patent Publication No. WO99/02657; Conzelmann, 1998, Ann. Rev. Genet., 32:123-162; Roberts and Rose,1998, Virol., 247:1-6; Lawson et al,1995 Proc. Natl. Acad Sci., USA 92:4477-4481. Additionally, for example, the genomic sequence of VSV (Indiana) is set out under Accession No. NC001560 in the NCBI database. Other sequences for VSV, including VSV (Chandipura) sequences are available in that database; for example, see Accession Nos. Ay382603, Af128868, V01208, V01207, V01206, M16608, M14715, M14720 and J04350, among others. VSV strains, such as New Jersey and Indiana, among others are also available from depositories such as the American Type Culture Collection, Rockville, Maryland (see, e.g., Accession Nos. VR-1238 and VR-1239). Other sequences are described or referenced in the documents cited throughout this specification.

VSV genomes have been shown to accommodate more than one foreign gene, with expansion to at least three kilobases. The genomes of these viruses are very stable, do not undergo recombination, and rarely incur significant mutations. In addition, since their replication is cytoplasmic, and their genomes are comprised of RNA, they are incapable of integrating within the genomes of infected host cells. Also, these negative-strand RNA viruses possess relatively simple transcriptional control sequences, which are readily manipulatable for efficient foreign gene insertion. Finally, the level of foreign gene expression can be modulated by changing the position of the foreign gene relative to the viral transcription promoter. The 3' to 5' gradient of gene expression reflects the decreasing likelihood that the transcribing viral polymerase will traverse successfully each intergenic gene stop/gene start signal encountered as it progresses along the genome template. Thus, foreign genes placed in proximity to a 3' terminal transcription initiation promoter are expressed abundantly, while those inserted in more distal genomic positions, are less so.

VSV replicates to high titers in a large array of different cell types, and viral proteins are expressed in great abundance. This not only means that VSV will act as a potent functional foreign gene delivery vehicle, but also, that relevant rVSV vectors can be scaled to manufacturing levels in cell lines approved for the production of human biologicals.

The rVSV has a remarkable capacity to deliver foreign genes encoding critical protective immunogens from viral pathogens to a broad array of different cell types, and to subsequently cause the abundant expression of authentically-configured immunogenic proteins (Haglund, K., et al, 2000 Virol., 268:112-21; Kahn, J. S. et al, 1999 Virol., 254:81-91; Roberts, A. et al, 1999 J. Virol., 73:3723-32; Rose, N. F. et al, 2000 J. Virol., 74:10903-10; and Schlereth, B. et al. 2000 J. Virol., 74:4652-7). The immunogens, so expressed, simultaneously elicit both highly durable virus neutralizing antibody responses, as well as protective cytotoxic T lymphocytes (CTL) (Roberts, A. et al, 1998 J. Virol, 72:4704-11).

In addition to rVSV's efficacy, this live virus gene delivery vehicle is safe, since wild-type VSV produces little to no disease symptoms or pathology in healthy humans, even in the face of substantial virus replication (Tesh, R. B. et al, 1969 Am. J. Epidemiol., 90:255-61). Additionally human infection with, and thus pre-existing immunity to VSV is rare. Given further attenuation, these rVSV compositions are suitable for use in immunocompromised or otherwise less robust human subjects. A significant advantage of use of the VSV vector in this method is that a number of serotypes of VSV exist due to the exchange or modification of the viral attachment protein G of the VSV. Thus, different serotypes of VSV vector carrying the same heterologous antigen can be used for repeated administration to avoid any interfering neutralizing antibody response generated to the VSV G protein by host's immune system.

A recombinant VSV can be designed using techniques previously described in the art, which carries the selected antigen and its regulatory sequences inserted into any position of the VSV under the control of the viral transcription promoter. In one embodiment, the heterologous gene encoding the selected antigen is inserted between the G and L coding regions of VSV. In another embodiment, the heterologous gene may be fused in the site of the G protein. In still other embodiments, the heterologous gene is fused to the site, or adjacent to, any of the other VSV genes.

In still other embodiments, the genes are 'shuffled' to different positions in the genome. In particular, the *N* gene is 'shuffled' to different positions in the genome. Cloning to produce the shuffled recombinant cDNA sequences involves modification of the original VSV plasmid backbone (pVSV-XN1). Three variants of this original vector include plasmids that deviate from the normal gene order (3'-*N-P-M-G-L-5')* as follows: i) *3'-P-N-M-G-L-5';* ii) *3'-P-M-N-G-L-5';* and iii) *3'-P-M-G-N-L-5'.* The cloning strategy used to create these plasmids employs a method described by Ball, L. A. et al. 1999 J. Virol., 73:4705-12. This technique takes advantage of the fact that the gene-end/gene-start signals found between each coding sequence are nearly identical, and allows gene rearrangements to be constructed without introducing any nucleotide substitutions. Alternatively, a few strategic point mutations may be introduced into noncoding sequences to create convenient restriction sites that facilitate genome rearrangements.

In still further embodiments of these vectors, the carboxy-terminal coding sequence for the 29 amino acid cytoplasmic domain of the *G* gene is truncated by deleting amino acids from the 5' C terminus of the *G* gene. Alternatively, the *G* gene is deleted entirely. In one embodiment, the entire cytoplasmic domain of the *G* gene is removed. In another embodiment at least 28 amino acids of the cytoplasmic domain are removed. In still a further embodiment, about 20 amino acids of the cytoplasmic domain are deleted. In still a further embodiment, about 10 or fewer amino acids of the cytoplasmic domain are deleted.

Both the shuffled genome approach and the G protein modification approach reportedly generate partial growth defects (Flanagan, E. B., et al 2001 J. Virol. 75:6107-14; Schnell, M. J. et al. 1998 EMBO J., 17:1289-96). It is anticipated that such modifications of the VSV may lead to a more attenuated phenotype or even to a non-replicating VSV for use in various embodiments of the present invention. See, e.g., Johnson, J. E. et al, 1998 Virol., 251:244-52.37; Johnson, J. E., et al., 1997 J. Virol., 71:5060-8.

Similarly, the selected antigen may be an antigen identified in the discussion below. In one embodiment of the immunogenic compositions herein, the selected antigen is an HIV-1 *gag* and/or *env (gp160)* gene of clade B virus isolate. In still other embodiments, the antigen is an HIV-1 *pol, nef, vpr, vpu, vif* or *tat* gene. Preferably the antigen sequence is optimized, such as by codon selection appropriate to the intended host and/or by removal of any inhibitory sequences, also discussed below with regard to antigen preparation.

To overcome any potential problem of diminished vector replication efficiencies with sequential administration, a vector set of similar design, each carrying a *G* gene from a different VSV serotype, permits successful booster immunizations. The primary amino acid sequences of the G proteins from VSV Indiana, New Jersey, and Chandipura, are sufficiently divergent such that preexisting immunity to one does not preclude infection and replication of the others. Thus, the neutralizing antibody response generated by rVSV (Indiana) should not interfere with replication of either rVSV (New Jersey) or rVSV (Chandipura). A vector set that can permit successful sequential immunizations can be prepared by replacing the G gene from VSV Indiana with either the divergent homolog from VSV Chandipura or from VSV New Jersey, forming three immunologically distinct vectors.

This rVSV immunogenic composition may include therefore one rVSV encoding a single selected antigen for expression in the host. According to the present method, the rVSV immunogenic composition comprises one rVSV comprising a nucleic acid sequence encoding more than one copy of the same selected antigen. Alternatively, the composition may contain one rVSV expressing multiple selected antigens. Each antigen may be under the control of separate regulatory elements or components. Alternatively, each antigen may be under the control of the same regulatory elements. In still another embodiment, the rVSV composition may contain multiple rVSVs, wherein each rVSV encodes the same or a different antigen.

In still a further embodiment, the rVSV immunogenic composition may further contain or be administered with, a cytokine, lymphokine or genetic adjuvant. The cytokine may be administered as a protein or in a plasmid as above-mentioned or be encoded by insertion of the cytokine encoding sequence in a recombinant VSV. For example, the cytokine encoding sequence may be inserted into any position in the VSV genome and expressed from the viral transcription promoter. A host of such suitable adjuvants for which nucleic acid sequences are available are identified below. In the embodiments exemplified in this invention, a desirable cytokine for administration with the rVSV composition of this invention is Interleukin-12.

The rVSV composition is desirably administered in a pharmaceutically acceptable diluent, excipient or carrier, such as those discussed below. Although the composition may be administered by any selected route of administration, in one embodiment a desirable method of administration is intranasal.

In one embodiment of the method of this invention, in which the rVSV composition is the boosting composition, the method includes administering at least one rVSV immunogenic composition after administration of a DNA immunogenic priming composition. The rVSV composition expresses the same antigen as expressed by the priming composition. In one embodiment, the rVSV composition includes an additional recombinant virus encoding a selected cytokine. In still another embodiment, the rVSV includes a sequence expressing a cytokine, e.g., IL-12 present in the same rVSV as is expressing the antigen. In still another embodiment, multiple rVSV compositions are administered as later boosters. In one embodiment at least two rVSV compositions are administered following the priming compositions. In another embodiment at least three rVSV compositions are administered following the priming compositions.

Desirably, as discussed above, each subsequent rVSV composition has a different serotype, but the same antigen encoding sequence. The different serotypes are selected from among known naturally occurring serotypes and from among any synthetic serotypes provided by manipulation of the VSV G protein. Among known methods for altering the G protein of rVSV are the technology described in International Publication No. WO99/3264 and Rose, N. F. et al. 2000 J. Virol, 74:10903-10.

In another embodiment, each rVSV has a different antigen encoding sequence, but the same VSV G protein. In still another embodiment, each rVSV has a different antigen encoding sequence, and a different VSV G protein. As detailed in the examples, one embodiment of a series ofrVSV boosting compositions comprises two optimized rVSVs containing an HIV-1 *gp160 env* gene, with one rVSV being the Indiana serotype and the other being the Chandipura serotype.

According to the present invention, this rVSV immunogenic composition may be administered as a boosting composition subsequent to the administration of the priming DNA immunogenic composition that presents the same antigen to the host. In any of the embodiments of the method of the invention, the second and any additional rVSV is administered as a booster following the first rVSV administration. The additional rVSV boosters in one embodiment are of the same serotype bearing the same antigen. Alternatively, the additional rVSV boosters having different serotypes are serially administered before administration of the boosting DNA immunogenic composition. It has been shown to be useful to administer at least three boosters. When used as a boosting composition, the rVSV compositions are administered serially, after the priming DNA immunogenic compositions. When used as the priming composition, the rVSV immunogenic composition is administered once or preferably multiple times. The additional rVSV boosters in one embodiment are of the same serotype bearing the same antigen. Alternatively, the additional rVSV boosters having different serotypes are serially administered before administration of the boosting DNA immunogenic composition.

Examples of suitable rVSV constructs that are capable of expressing an HIV-1 protein *in vivo* are described in detail in the following examples, and in the following publications, e.g., Rose et al, 2000 Virol, 268:112-121; Rose et al, 2000 J. Virol., 74-10903-10910; Rose et al 2001 Cell, 106:539-549; Rose et al, 2002 J. Virol., 76:2730-2738; Rose et al, 2002 J. Virol., 76:7506-7517. Additional rVSV vectors may be further attenuated, either by progressive truncations of the VSV G protein, or by VSV structural gene shuffling as mentioned above. Non-replicating VSV may also be used according to this invention. rVSVs displaying a desired balance of attenuation and immunogenicity are anticipated to be useful in this invention.

Several illustrative rVSV constructs exemplified in the Examples below have the recombinant genomes:
(1) 3' N- P- M- G-HIV *env*-L-5'
(2) 3' N- P- M- G-HIV *gag*-L-5'
(3) 3' N- P- M- G-SIV *gag-*L-5'.
In one embodiment, a method and immunogenic composition of this invention employs one of these rVSV constructs. In another embodiment, a method and immunogenic composition of this invention employ both an rVSV-HIV *env* and an rVSV-HIV *gag*. This latter immunogenic composition simultaneously elicits potent humoral immune responses to authentically configured gp160, plus CTL (to Env and Gag) capable of killing HIV-1 virus infected cells. The HIV-1 gp160 expressed in this manner binds to CD4/co-receptor and undergoes the conformational changes associated with receptor binding. Proper gp160/ receptor interactions expose the cryptic virus neutralizing determinants present on gp160 which are required for the induction of antibody-mediated protection from infection (see, e.g., LaCasse, R. A. et al, 1999 Science. 283:357-62).

### C. Antigens for Use in the Immunogenic Compositions of this Invention

The antigenic or immunogenic compositions useful in the methods and compositions of this invention enhance the immune response in a vertebrate host to a selected antigen. The selected antigen, when expressed by the plasmid DNA or VSV, may be a protein, polypeptide, peptide, fragment or a fusion thereof derived from a pathogenic virus, bacterium, fungus or parasite. Alternatively, the selected antigen may be a protein, polypeptide, peptide, fragment or fusion thereof derived from a cancer cell or tumor cell. In another embodiment, the selected antigen may be a protein, polypeptide, peptide, fragment or fusion thereof derived from an allergen so as to interfere with the production of IgE so as to moderate allergic responses to the allergen. In still another embodiment, the selected antigen may be a protein, polypeptide, peptide, fragment or fusion thereof derived from a molecule or portion thereof which represents those produced by a host (a self molecule) in an undesired manner, amount or location, such as those from amyloid precursor protein, so as to prevent or treat disease characterized by amyloid deposition in a vertebrate host. In one embodiment of this invention, the selected antigen is a protein, polypeptide, peptide or fragment derived from HIV-1.

The invention is also directed to methods for increasing the ability of an immunogenic composition containing a selected antigen (1) from a pathogenic virus, bacterium, fungus or parasite to elicit the immune response of a vertebrate host, or (2) from a cancer antigen or tumor-associated antigen from a cancer cell or tumor cell to elicit a therapeutic or prophylactic anti-cancer effect in a vertebrate host, or (3) from an allergen so as to interfere with the production of IgE so as to moderate allergic responses to the allergen, or (4) from a molecule or portion thereof which represents those produced by a host (a self molecule) in an undesired manner, amount or location, so as to reduce such an undesired effect.

In another embodiment, desirable viral immunogenic compositions utilizing the prime/boost regimen according to the present disclosure include those directed to the prevention and/or treatment of disease caused by, without limitation, Human immunodeficiency virus, Simian immunodeficiency virus, Respiratory syncytial virus, Parainfluenza virus types 1-3, Influenza virus, Herpes simplex virus, Human cytomegalovirus, Hepatitis A vitus, Hepatitis B virus, Hepatitis C virus, Human papillomavirus, Poliovirus, rotavirus, caliciviruses, Measles virus, Mumps virus, Rubella virus, adenovirus, rabies virus, canine distemper virus, rinderpest virus, Human metapneumovirus, avian pneumovirus (formerly turkey rhinotracheitis virus), Hendra virus, Nipah virus, coronavirus, parvovirus, infectious rhinotracheitis viruses, feline leukemia virus, feline infectious peritonitis virus, avian infectious bursal disease virus, Newcastle disease virus, Marek's disease virus, porcine respiratory and reproductive syndrome virus, equine arteritis virus and various Encephalitis viruses.

In another embodiment, desirable bacterial immnunogenic compositions utilizing the prime/boost regimen of the present disclosure include those directed to the prevention and/or treatment of disease caused by, without limitation, *Haemophilus influenzae* (both typable and nontypable), *Haemophilus somnus, Moraxella catarrhalis, Streptococcus pneumoniae, Streptococcus pyogenes, Streptococcus agalactiae, Streptococcus faecalis, Helicobacter pylori, Neisseria meningitidis, Neisseria gonorrhoene, Chlamydia trachomatis, Chlamydia pneumoniae, Chlamydia psittaci, Bordetella pertussis, Alcoiccoccus atiditis, Salmonella typhi, Salmonella typhimurium, Salmonella choleraesuis, Escherichia coli, Shigella, Vibrio cholerae, Corynebacterium diphtheriae, Mycobacterium tuberculosis, Mycobacterium avium-Mycobacterium intracellulare complex, Proteus mirabilis, Proteus vulgaris. Staphylococcus aureus, Staphylococcus epidermidis, Clostridium tetani, Leptospira interrogans, Borrelia burgdorferi, Pasteurella haemolytica, Pasteurella multocida, Actinobacilhus pleuropneumoniae and Mycoplasma gallisepticum.*

In another embodiment,desirable immunogenic compositions against fungal pathogens utilizing the prime/boost regimen according to the present disclosure include those directed to to the prevention and/or treatment of disease caused by, without limitation, *Aspergillis, Blastomyces, Candida, Coccidiodes, Cryptococcus and Histoplasma.*

In another embodiment, desirable immunogenic compositions against parasites utilizing the prime/boost regimen according to the present disclosure include those directed to the prevention and/or treatment of disease caused by, without limitation, *Leishmania major, Ascaris, Trichuris, Giardia, Schistosoma, Cryptosporidium, Trichomonas, Toxoplasma gondii and Pneumocystis carinii.*

In another embodiment, desirable immunogenic compositions for eliciting a therapeutic or prophylactic anti-cancer effect in a vertebrate host, which utilise the prime/boost regimen according to the present disclosure include those utilizing a cancer antigen or tumor-associated antigen, including, without limitation, prostate specific antigen, carcino-embryonic antigen, MUC-1, Her2, CA-125 and MAGE-3.

Nucleotide and protein sequences for the above-listed, known antigens are readily publicly available through databases such as NCBI, or may be available from other sources such as the American Type Culture Collection and universities.

Desirable immunogenic compositions for moderating responses to allergens in a vertebrate host, which utilize the prime/boost regimen of this invention include those containing an allergen or fragment thereof. Examples of such allergens are described in United States Patent No.5,830,877 and International Patent Publication No: WO99/51259. Such allergens include, without limitation, pollen, insect venoms, animal dander, fungal spores and drugs (such as penicillin). These immunogenic compositions interfere with the production of IGB antibodies, a known cause of allergic reactions.

Desirable immunogenic compositions for moderating responses to self molecules in a vertebrate host, which utilize the prime/boost regimen according to the present disclosure include those containing a self molecule or fragment thereof. Examples of such self molecules include the β-chain insulin involved in diabetes, the G17 molecule involved in gastroesophageal reflux disease, and antigens which down regulate autoimmune responses in diseases such as multiple sclerosis, lupus and rheumatoid arthritis. Also included is the β-amyloid peptide (also referred to as Aβ peptide), which is an internal, 39-43 amino acid fragment of amyloid precursor protein (APP), which is generated by processing of APP by the β and γ secretase enzymes. The Aβ1-42 peptide has the following sequence SEQ ID NO:1: Asp Ala Gln Phe Arg His Asp Ser Gly Tyr Gln Val His His Gln Lys Len Val Phe Phe Ala Glu Asp Val Gly Ser Asn Lys Gly Ala Ile Ile Gly Leu Met Val Gly Gly Val Val Ile Ala.

It is also desirable in selection and use of the antigenic sequences for design of the DNA plasmids and rVSV constructs of this invention to alter codon usage of the selected antigen-encoding gene sequence, as well as the DNA plasmids into which they are inserted, and/or to remove inhibitory sequences therein. The removal of inhibitory sequences can be accomplished by using the technology discussed in detail in US Patent Nos. 5,965,726; 5.972,596; 6,174,666; 6.291,664; and 6,414,132; and in International Patent Publication No. WO01/46408. Briefly described, this technology involves mutating identified inhibitor/instability sequences in the selected gene, preferably with multiple point mutations.

As one specific embodiment exemplified below, the immunogenic plasmid and rVSV compositions of this invention desirably employ one or more sequences optimized to encode HIV-1 antigens,such as the gag, pol and nef antigens, or immunogenic fragments or fusions thereof. The *gag* and *env* genes of the chimeric simian-human immunodeficiency virus (SHIV) (89.6P) are useful to make rVSVs, such that protection from infection and mitigation of virus burden and disease can be demonstrated in a Rhesus macaque model of disease. The examples below demonstrate use of the SHIV analogs, i.e. SIV *gag* and HIV 89.6P *env.*

### D. Promoters Useful in the Immunogenic DNA Plasmid or rVSV Constructs

Suitable promoters for use in any of the components of this invention may be readily selected from among constitutive promoters, inducible promoters, tissue-specific promoters and others. Examples of constitutive promoters that are nonspecific in activity and employed in the nucleic acid molecules encoding an antigen of this invention include, without limitation, the retroviral Rous sarcoma virus (RSV) promoter, the retroviral LTR promoter (optionally with the RSV enhancer), the cytomegalovirus (CMV) promoter (optionally with the CMV enhancer) (see, e.g., Boshart et al, 1985 Cell, 41:521-530), the SV40 promoter, the dihydrofolate reductase promoter, the β-actin promoter, the phosphoglycerol kinase (PGK) promoter, and the EF1α promoter (Invitrogen). Inducible promoters that are regulated by exogenously supplied compounds, include, without limitation, the arabinose promoter, the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al, 1996 Proc. Natl. Acad. Sci. USA, 93:3346-3351), the tetracycline-repressible system (Gossen et al, 1992 Proc. Natl. Acad. Sci. USA, 89:5547-5551), the tetracycline-inducible system (Gossen et al, 1995 Science, 268:1766-1769, see also Harvey et al, 1998 Curr. Opin. Chem. Biol., 2:512-518), the RU486-inducible system (Wang et al, 1997 Nat. Biotech., 15:239-243 and Wang et al, 1997 Gene Ther., 4:432-441) and the rapamycin-inducible system (Magari et al, 1997 J. Clin. Invest., 100: 2865-2872).

Other types of inducible promoters that may be useful in this context are those regulated by a specific physiological state, e.g., temperature or acute phase or in replicating cells only. Useful tissue-specific promoters include the promoters from genes encoding skeletal β-actin, myosin light chain 2A, dystrophin, muscle creatine kinase, as well as synthetic muscle promoters with activities higher than naturally-occurring promoters (see Li et al., 1999 Nat. Biotech., 17:241-245). Examples of promoters that are tissue-specific are known for the liver (albumin, Miyatake et al. 1997 J. Virol., 71:5124-32; hepatitis B virus core promoter, Sandig et al., 1996 Gene Ther., 3: 1002-9; alpha-fetoprotein (AFP), Arbuthnot et al., 1996 Hum. Gene Ther., 7:1503-14), bone (osteocalcin, Stein et al., 1997 Mol. Biol. Rep., 24:185-96; bone sialoprotein, Chen et al., 1996 J. Bone Miner. Res., 11:654-64), lymphocytes (CD2, Hansal et al., 1988 J. Immunol., 161:1063-8; immunoglobulin heavy chain; T cell receptor α chain), neuronal (neuron-specific enolase (NSE) promoter, Andersen et al. 1993 Cell. Mol.Neurobiol., 13:503-15; neurofilament light-chain gene, Piccioli et al., 1991 Proc. Natl. Acad. Sci. USA, 88:5611-5; the neuron-specific *ngf* gene, Piccioli et al., 1995 Neuron, 15:373-84); among others. See, e.g., International Patent Publication No. WO00/55335 for additional lists of known promoters useful in this context.

### E. Carriers, Excipients, Adjuvants and Formulations Useful for the Immunogenic Compositions of this Invention

The immunogenic compositions useful in this invention, whether the DNA plasmid or rVSV compositions, further comprise an immunologically acceptable diluent or a pharmaceutically acceptable carrier, such as sterile water or sterile isotonic saline. The antigenic compositions may also be mixed with such diluents or carriers in a conventional manner. As used herein the language "pharmaceutically acceptable carrier" is intended to include any and all solvents, dispersion media, coatings, antibacterial and antifungal agents, isotonic and absorption delaying agents, and the like, compatible with administration to humans or other vertebrate hosts. The appropriate carrier is evident to those skilled in the art and will depend in large part upon the route of administration.

Still additional components that may be present in the immunogenic compositions of this invention are adjuvants, preservatives, surface active agents, and chemical stabilizers, suspending or dispersing agents. Typically, stabilizers, adjuvants, and preservatives are optimized to determine the best formulation for efficacy in the target human or animal.

### 1. Adjuvants

An adjuvant is a substance that enhances the immune response when administered together with an immunogen or antigen. A number of cytokines or lymphokines have been shown to have immune modulating activity, and thus may be used as adjuvants, including, but not limited to, the interleukins 1-α, 1-β, 2,4,5,6,7, 8,10,12 (see, e.g., U.S. Patent No. 5,723,127),13,14,15,16,17 and 18 (and its mutant forms), the interferons-α,β and γ, granulocyte-macophage colony stimulating factor (see, e.g., U.S. Patent No. 5,078,996 and ATCC Accession Number 39900), macrophage colony stimulating factor, granulocyte colony stimulating factor, GSF, and the tumor necrosis factors α and β. Still other adjuvants useful in this invention include a chemokine, including without limitation, MCP-1, MIP-1α, MIP-1β, and RANTES. Adhesion molecules, such as a selectin, e.g., L-selectin, P-selectin and E-selectin may also be useful as adjuvants. Still other useful adjuvants include, without limitation, a mucin-like molecule, e.g., CD34, GlyCAM-1 and MadCAM-1, a member of the integrin family such as LFA-1, VLA-1, Mac-1 and p150.95, a member of the immunoglobulin superfamily such as PECAM, ICAMs, e.g., ICAM-1, ICAM-2 and ICAM-3, CD2 and LFA-3, co-stimulatory molecules such as CD40 and CD40L,growth factors including vascular growth factor, nerve growth factor, fibroblast growth factor, epidermal growth factor, B7.2, PDGF, BL-1, and vascular endothelial growth factor, receptor molecules including Fas, TNF receptor, Flt, Apo-1, p55, WSL-1, DR3, TRAMP, Apo-3, AIR, LARD, NGRF, DR4, DR5, KILLER, TRAIL-R2, TRICK2, and DR6. Still another adjuvant molecule includes Caspase (ICE). See, also International Patent Publication Nos. WO98/17799 and WO99/43839.

Suitable adjuvants used to enhance an immune response include, without limitation, MPL™ (3-O-deacylated monophosphoryl lipid A; Corixa, Hamilton, MT), which is described in U.S. Patent No. 4,912,094. Also suitable for use as adjuvants are synthetic lipid A analogs or aminoalkyl glucosamine phosphate compounds (AGP), or derivatives or analogs thereof, which are available from Corixa (Hamilton. MT), and which are described in United States Patent No. 6,113,918. One such AGP is 2-[(R)-3-Tetradecanoyloxytetradecanoylamino]ethyl 2-Deoxy-4-O-phosphono-3-O-[(R)-3-tetradecanoyoxytetradecanoyl]-2-[(R)-3-tetradecanoyloxytetradecanoyl-amino]-b-D-glucopyranoside, which is also known as 529 (formerly known as RC529). This 529 adjuvant is formulated as an aqueous form or as a stable emulsion.

Still other adjuvants include mineral oil and water emulsions, aluminum salts (alum), such as aluminum hydroxide, aluminum phosphate, etc., Amphigen, Avridine, L121/squalene, D-lactide-polylactide/glycoside, pluronic polyols, muramyl dipeptide, killed *Bordetella,* saponins, such as Stimulon™ QS-21 (Antigenics, Framingham, MA.), described in U.S. Patent No. 5,057,540, and particles generated therefrom such as ISCOMS (immunostimulating complexes), *Mycobacterium tuberculosis,* bacterial lipopolysaccharides, synthetic polynucleotides such as oligonucleotides containing a CpG motif (U.S. Patent No. 6,207,646, a pertussis toxin (PT), or an *E. coli* heat-labile toxin (LT), particularly LT-K63, LT-R72, PT-K9/G129; see, e.g., International Patent Publication Nos. WO 93/13302 and WO 92/19265.

Also useful as adjuvants are cholera toxins and mutants thereof, including those described in published International Patent Application number WO 00/18434 (wherein the glutamic acid at amino acid position 29 is replaced by another amino acid (other than aspartic acid), preferably a histidine). Similar CT toxins or mutants are described in published International Patent Application number WO 02/098368 (wherein the isoleucine at amino acid position 16 is replaced by another amino acid, either alone or in combination with the replacement of the serine at amino acid position 68 by another amino acid; and/or wherein the valine at amino acid position 72 is replaced by another amino acid). Other CT toxins are described in published International Patent Application number WO 02/098369 (wherein the arginine at amino acid position 25 is replaced by another amino acid; and/or an amino acid is inserted at amino acid position 49; and/or two amino acids are inserted at amino acid positions 35 and 36).

In one embodiment exemplified below, the desired adjuvant is IL-12, which is expressed from a plasmid. See, e.g., US Patent Nos. 5,457,038; 5,648,467; 5,723,127 and 6,168,923. This IL-12 expressing plasmid is incorporated into the immunogenic DNA plasmid-containing priming composition of the examples. However, it should be noted that this plasmid could be administered to the mammalian or vertebrate host with the rVSV composition (or expressed by the rVSV) or alone, between the priming and boosting compositions. In one embodiment, the cytokine may be administered as a protein. In a preferred embodiment, the cytokine is administered as a nucleic acid composition comprising a DNA sequence encoding the cytokine under the control of regulatory sequences directing expression thereof in a mammalian cell. In still another useful embodiment the cytokine-expressing plasmid is administred with the DNA composition. In still another embodiment, the cytokine is administered between the administrations of the priming composition and the boosting composition. In yet another step, the cytokine is administered with the boosting step. In still another embodiment, the cytokine is administered with both priming and boosting compositions.

Where the priming composition is the DNA plasmid composition, as in the examples below, the plasmid composition can comprise a DNA sequence encoding the cytokine under the control of regulatory sequences directing expression thereof in the mammalian cell. In some embodiments, the cytokine-encoding sequence is present on the same DNA plasmid as the antigen-encoding sequence. In still other embodiments, the cytokine-encoding sequence is present on a DNA plasmid different from the DNA plasmid encoding the antigen.

### 2. Facilitating Agents or Co-Agents

In addition to a carrier as described above, immunogenic compositions composed of polynucleotide molecules desirably contain optional polynucleotide facilitating agents or "co-agents", such as a local anesthetic, a peptide, a lipid including cationic lipids, a liposome or lipidic particle, a polycation such as polylysine, a branched, three-dimensional polycation such as a dendrimer, a carbohydrate, a cationic amphiphile, a detergent, a benzylammonium surfactant, or another compound that facilitates polynucleotide transfer to cells. Such a facilitating agent includes the local anesthetic bupivacaine or tetracaine (see U.S. Patent Nos. 5,593,972; 5,817,637; 5,380,876; 5,981,505 and 6,383,512 and International Patent Publication No. WO98/17799). Other non-exclusive examples of such facilitating agents or co-agents useful in this invention are described in U. S. Patent Nos. 5,703,055; 5,739,118; 5,837,533; International Patent Publication No. WO96/10038, published April 4,1996; and International Patent Publication No WO94/16737, published August 8,1994.

Most preferably, the local anesthetic is present in an amount that forms one or more complexes with the nucleic acid molecules. When the local anesthetic is mixed with nucleic acid molecules or plasmids of this invention, it forms a variety of small complexes or particles that pack the DNA and are homogeneous. Thus, in one embodiment of the immunogenic compositions of this invention, the complexes are formed by mixing the local anesthetic and at least one plasmid of this invention. Any single complex resulting from this mixture may contain a variety of combinations of the different plasmids. Alternatively, in another embodiment of the compositions of this invention, the local anesthetic may be premixed with each plasmid separately. The separate mixtures are then combined in a single composition to ensure the desired ratio of the plasmids is present in a single immunogenic composition, if all plasmids are to be administered in a single bolus administration. Alternatively, the local anesthetic and each plasmid may be mixed separately and administered separately to obtain the desired ratio.

Where, hereafter, the term "complex" or "one or more complexes" or "complexes" is used to define this embodiment of the immunogenic composition, it is understood that the term encompasses one or more complexes. Each complex contains a mixture of the plasmids, or a mixture of complexes formed discretely. Each complex can contain only one type of plasmid or complex, or a mixture of plasmids or complexes, wherein each complex contains a polycistronic DNA. Preferably, the complexes are between about 50 to about 150 mm in diameter. When the facilitating agent used is a local anesthetic, preferably bupivacaine, an amount from about 0.1 weight percent to about 1.0 weight percent based on the total weight of the polynucleotide composition is preferred. See, also, International Patent Publication No. WO99/21591, and which teaches the incorporation of benzylammonium surfactants as co-agents, preferably administered in an amount between about 0.001-0.03 weight %. According to the present invention, the amount of local anesthetic is present in a ratio to said nucleic acid molecules of 0.01-2.5% w/v local anesthetic to 1-10 µg/ml nucleic acid. Another such range is 0.05-1.25% w/v local anesthetic to 100 µg/ml to 1 mg/ml nucleic acid.

### 3. Other Additives to the Immunogenic Compositions

Other additives can be included in the immunogenic compositions of this invention, including preservatives, stabilizing ingredients, surface active agents, and the like.

Suitable exemplary preservatives include chlorobutanol, potassium sorbate, sorbic acid, sulfur dioxide, propyl gallate, the parabens, ethyl vanillin, glycerin, phenol, and parachlorophenol.

Suitable stabilizing ingredients that may be used include, for example, casamino acids, sucrose, gelatin, Phenol red, N-Z amine, monopotassium diphosphate, lactose, lactalbumin hydrolysate, and dried milk.

Suitable surface active substances include, without limitation, Freunds incomplete adjuvant, quinone analogs, hexadecylamine, octadecylamine, octadecyl amino acid esters, lysolecithin, dimethyl-dioctadecylammonium bromide), methoxyhexadecylgylcerol, and pluronic polyols; polyamines, e.g., pyran, dextransulfate, poly IC, carbopol; peptides, e.g., muramyl peptide and dipeptide, dimethylglycine, tuftsin; oil emulsions; and mineral gels, e.g., aluminum phosphate, etc. and immune stimulating complexes (ISCOMS). The plasmids and rVSVs may also be incorporated into liposomes for use as an immunogenic composition. The immunogenic compositions may also contain other additives suitable for the selected mode of administration of the composition. The composition of the invention may also involve lyophilized polynucleotides, which can be used with other pharmaceutically acceptable excipients for developing powder, liquid or suspension dosage forms. See, e.g., Remington: The Science and Practice of Pharmacy, VoL 2, 19th edition (1995), e.g., Chapter 95 Aerosols; and International Patent Publication No. WO99/45966.

These immunogenic compositions can contain additives suitable for administration via any conventional route of administration. In some preferred embodiments, the immunogenic composition of the invention is prepared for administration to human subjects in the form of, for example, liquids, powders, aerosols, tablets, capsules, enteric-coated tablets or capsules, or suppositories. Thus, the immunogenic compositions may also include, but are not limited to, suspensions, solutions, emulsions in oily or aqueous vehicles, pastes, and implantable sustained-release or biodegradable formulations. In one embodiment of a formulation for parenteral administration, the active ingredient is provided in dry (*i.e*., powder or granular) form for reconstitution with a suitable vehicle (*e.g*., sterile pyrogen-free water) prior to parenteral administration of the reconstituted composition. Other useful parenterally-administrable formulations include those which comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

The immunogenic compositions of the present invention, are not limited by the selection of the conventional, physiologically acceptable, carriers, adjuvants, or other ingredients useful in pharmaceutical preparations of the types described above. The preparation of these pharmaceutically acceptable compositions, from the above-described components, having appropriate pH isotonicity, stability and other conventional characteristics is within the skill of the art.

### F. Dosages and Routes of Administration for Immunogenic Compositions of the Present Invention

In general, selection of the appropriate "effective amount" or dosage for the components of the immunogenic composition(s) of the present invention will also be based upon the identity of the antigen in the immunogenic composition(s) employed, as well as the physical condition of the subject, most especially including the general health, age and weight of the immunized subject. The method and routes of administration and the presence of additional components in the immunogenic compositions may also affect the dosages and amounts of the plasmid and rVSV compositions. Such selection and upward or downward adjustment of the effective dose is within the skill of the art. The amount of plasmid and rVSV required to induce an immune response, preferably a protective response, or produce an exogenous effect in the patient without significant adverse side effects varies depending upon these factors. Suitable doses are readily determined by persons skilled in the art.

The antigenic or immunogenic compositions of this invention are administered to a human or to a non-human vertebrate by a variety of routes including, but not limited to, intranasal, oral, vaginal, rectal, parental, intradermal, transdermal (*see,* e.g., International patent publication No. WO 98/20734), intramuscular, intraperitoneal, subcutaneous, intravenous and intraarterial. The appropriate route is selected depending on the nature of the immunogenic composition used, and an evaluation of the age, weight, sex and general health of the patient and the antigens present in the immunogenic composition, and similar factors by an attending physician.

In the examples provided below, the immunogenic DNA compositions are administered intramuscularly (i.m.). In one embodiment, it is desirable to administer the rVSV compositions intranasally, rather than im. However, the selection of dosages and routes of administration are not limitations upon this invention.

Similarly, the order of immunogenic composition administration and the time periods between individual administrations may be selected by the attending physician or one of skill in the art based upon the physical characteristics and precise responses of the host to the application of the method. Such optimization is expected to be well within the skill of the art.

### G. Kit Components

In still another embodiment, the present invention provides a pharmaceutical kit for ready administrations of an immunogenic, prophylactic, or therapeutic regimen for treatment of any of the above-noted diseases or conditions for which an immune response to an antigen is desired. This kit is designed for use in a method of inducing a high level of antigen-specific immune response in a mammalian or vertebrate subject. The kit contains at least one immunogenic composition comprising a DNA plasmid comprising a DNA sequence encoding an antigen under the control of regulatory sequences directing expression thereof in a mammalian or vertebrate cell. Preferably multiple prepackaged dosages of the DNA immunogenic composition are provided in the kit for multiple administrations. The kit also contains at least one immunogenic composition comprising a replication competent, recombinant vesicular stomatitis virus (rVSV) comprising a nucleic acid sequence encoding the same antigen under the control of regulatory sequences directing expression thereof in a mammalian or vertebrate cell. Preferably multiple prepackaged dosages of the rVSV immunogenic composition are provided in the kit for multiple administrations.

Where the above-described immunogenic compositions do not also contain DNA plasmids and/or rVSV that express a cytokine, such as IL-12, the kit also optionally contains a separate cytokine composition or multiple prepackaged dosages of the cytokine composition for multiple administrations. These cytokine compositions are generally nucleic acid compositions comprising a DNA sequence encoding the selected cytokine under the control of regulatory sequences directing expression thereof in a mammalian or vertebrate cell.

The kit also contains instructions for using the immunogenic compositions in a prime/boost method as described herein. The kits may also include instructions for performing certain assays, various carriers, excipients, diluents, adjuvants and the like above-described, as well as apparatus for administration of the compositions, such as syringes, spray devices, etc. Other components may include disposable gloves, decontamination instructions, applicator sticks or containers, among other compositions.

In order that this invention may be better understood, the following examples are set forth. The examples are for the purpose of illustration only and are not to be construed as limiting the scope of the invention. All documents, publications and patents cited in the following examples are incorporated by reference herein.

As demonstrated in the Examples below, a prime/boost protocol of this invention induces in the immunized subject a surprising synergistic effect on antigen-specific cellular and humoral immune responses. In fact, when these responses induced by a prime/boost protocol of this invention are compared to the results of administering multiple priming compositions only or multiple boosting compositions only, the synergistic nature of the response to the compositions of this invention is dramatically evident. The combination of the presentation of the desired antigen by a DNA plasmid administration followed by a rVSV boost produces an increase in antigen-specific T cells in the immunized subject, that is considerably in excess of any additive response. Similarly, the increase demonstrated in the humoral response to the desired antigen is unexpectedly high with the use of both the DNA plasmid and rVSV immunogenic compositions in an immunization protocol. See, for example, Table 1 below and FIGS. 4 and 5.

### EXAMPLE 1: PREPARATION OF DNA PLASMIDS

This example describes illustrative plasmids useful in one embodiment of this invention as set out in Examples 2 and 3. These plasmids are not a limitation on the present invention, but have been optimized for use in the subsequent experiments. The following DNA immunogenic compositions were designed utilizing standard recombinant DNA techniques. The DNA backbone vector expressing HIV or SIV *gag* genes utilizes the HCMV promoter, BGH poly A termination sequence, the ColE1 bacterial origin of replication (ori); and a kanamycin resistance gene for selection.

### A. SIV gag p37 DNA. Plasmid

Plasmid WLV102 is a bacterial plasmid expressing as the selected antigen against which an immune response was desired, the SIV gag p37. Plasmid WLV102 (4383bp) consists of an RNA optimized truncated *gag* gene (p37) from SIV (Qiu et al, 1999 J. Virol, 73:9145-9152) inserted into the DNA plasmid expression vector WLV001. The *gag* gene was RNA optimized by inactivating the inhibitory sequences, thus allowing high level Rev independent expression of *gag* gene, using the technology discussed in detail in US Patent Nos. 5,965,726; 5,972,596; 6,174,666; 6,291,664; and 6,414,132; and in International Patent Publication No. WO01/46408.

The WLV102 plasmid backbone consists of three genetic units. The first is a eukaryotic gene expression unit that contains genetic elements from the HCMV immediate early promoter/enhancer (Boshart *et al*. **1985**, cited above) and the BGH polyadenylation signal (Goodwin EC and Rottman FM, 1982 J. Biol. Chem. 267: 16330-16334). The *gag* gene is cloned between SalI and EcoRI sites. The second component is a chimeric kanamycin resistance gene (*km'*) gene, adenyl 4'-nucleotidyl transferase type la (see United States Patent No. 5,851,804). This gene has been devised to confer resistance to limited number of aminoglycosides while it enables selection of bacteria containing the plasmid. The third component is a ColE1 bacterial origin of replication that is required for the propagation of the plasmid during fermentation of bacteria. This plasmid is illustrated in FIG. 1A.

### B. Plasmids Encoding a Cytokine - rIL-12 DNA

The Rhesus IL-12 WLV 104 plasmid is a dual promoter construct expressing the heterodimeric form of rhesus IL-12. Plasmid WLV103 is a dual promoter expression plasmid consisting of two genes encoding human IL12 proteins p35 and p40. The plasmid has a total of 6259 nucleotides. Each cistron in WLV103 containing one of the two interleukin 12 subunits, p35 or p40, is under the control of separate regulatory elements. The p35 subunit is under the control of HCMV promoter/enhancer, and the SV40 polyadenylation signal (cloned between SalI and MluI sites). The p40 subunit is under the control of the SCMV promoter and has a BGH polyadenylation signal (cloned into XhoI site).

The plasmid backbone consists of several components. The first is a eukaryotic gene expression unit that contains genetic elements from the HCMV immediate early promoter/enhancer and SV 40 polyadenylation signal (Fitzgerald M and Shenk T., 1981 Cell, 24: 251-260). The second component is a eukaryotic gene expression unit, composed of the SCMV promoter (Jeang et al. 1987 J. Virol., 61: 1559-1570) and the BGH polyadenylation signal. The third component is a chimeric kanamycin resistance gene (*km^{r}*) gene, adenyl 4'-nucleotidyl transferase type1a. The fourth component is a ColE1 bacterial origin of replication that is required for the propagation of the plasmid in bacteria.

The resulting plasmid vectors SIV gag/HIV gag and IL-12 DNA were analyzed by restriction enzyme digest. The plasmids were transiently transfected in Rhabdosarcoma cells grown in DMEM+10% fetal calf serum (FCS) medium and antibiotics. These cells were then analyzed for appropriate expression of the viral proteins using a specific monoclonal antibody for HIV gag (ABI) and polyclonal serum from rhesus (from SIV infected animals) for SIV gag in a Western Blot assay. IL-12 was detected in the supernatant with ELISA kits (R&D Systems) that detect p70 protein.

The plasmid vectors were expanded in transformed DH10B cells grown in LB medium supplemented with kanamycin, and were purified using the Qiagen kit according to manufacturer's specifications. The DNA was then analyzed by electrophoresis on an agarose gel against a known standard.

For use in the following experiments each plasmid was formulated at a concentration of 2.5 mg/mL in 0.25% bupivacaine to a total volume of 4.0 cc.

### EXAMPLE 2: PREPARATION OF RECOMBINANT VSV VECTORS

### A. VSV genomic cDNA cloning.

The genetic background for VSV genomic cDNA manipulation is pVSV-XN1 (Schnell, M. J., et al 1996 J. Virol., 70:2318-23). This clone contains a modified form of the VSV Indiana strain (VSVᵢ) cDNA sequence. The modifications include the addition of two unique restriction endonuclease recognition sites (XhoI and NheI), and added copies of VSV gene-start and VSV gene-end signals. When foreign genes such as HIV-1_{89.6p} *env gp160* or SIV *gag p55* or HIV-1 *gag,* are conveniently inserted between the XhoI and NheI sites, they reside in a position suitable for expression controlled by VSV transcriptional control signals. Also, the VSV cDNA sequence is flanked by cis-acting DNA sequences required to promote rescue of the live virus replicates. The T7 RNA polymerase promoter directs transcription of a primary transcript across the viral cDNA. The ribozyme cleaves the primary transcript to form the end of the RNA genome after T7 RNA polymerase terminates transcription.

Initially, the rVSVᵢ genomic clone (pVSV-XN1) was modified by insertion of the *gag* gene (HIV Clade B) between the *G* and *L* genes to produce plasmid prVSVi-*gag*. Similarly, a separate rVSVᵢ cDNA clone was made that contains the HIV *env* gene (genomic clone prVSVi-env). The *gag* and *env* cDNA sequences were prepared for insertion into pVSV-XN1 by amplifying the coding region sequences from plasmid templates that contain the HIV HXBc2 *gag* gene or HIV 6101 strain *env* gene. The primers used for PCR amplification contained terminal restriction enzyme cleavage sites appropriate for subsequent cloning; the 5' primer contained an XhoI site and the 3' primer contained an NheI site. The amplified coding region sequences were separately inserted into pVSV-XN1 to generate a clone containing *gag* and a clone containing *env.*

The *env* gene inserted into pVSV-XN1 was a modified form that encodes an HIV Env/VSV G fusion protein. Cell surface expression of Env after infection with a rVSV-HIV Env vector was shown to be enhanced if the cytoplasmic tail of Env was replaced by the shorter cytoplasmic tail of the VSV G protein using an overlap PCR procedure (see, e.g., Johnson, *et al*, **1998** cited above; Johnson *et al*., **1997**, cited above). The vector backbone of these two plasmids was altered by changing the Indiana *G* gene to that of the Chandipura or New Jersey serotypes according to published techniques. Exchanging *G* genes was accomplished by taking advantage of a unique MluI site in the *M* gene and the XhoI site that was engineered in the VSV cDNA clones.

VSV *G* gene coding sequences from the Chandipura strain or the New Jersey strain were PCR amplified using a 5' primer that extends across the MluI site within the *M* gene. A 3' PCR primer was used that contains sequences homologous to the 3' end of the *G* gene as well as additional terminal sequences corresponding to the gene-end/gene-start signal and the XhoI site. The *G* gene coding sequences amplified with these primers were used to replace the original Indiana *G* gene after it had been excised from the plasmid backbone with MluI and XhoI.

### B. Rescue of rVSV front cDNA Clones

The genomic cDNA plasmid or RNA transcribed from a genomic cDNA plasmid was not sufficient to initiate the viral replicative cycle after being introduced into a cell. By itself, viral genomic RNA was not an active template for translation or replication. Thus, recombinant virus must be recovered from the various VSV genomic cDNA constructs. Rescue procedures known in the art have made it possible to recover virus from cloned DNAs. Successful virus rescue required that VSV genomic RNA was present in the cell along with VSV N protein to encapsidate the viral genomic RNA, as well as P and L proteins, which form the viral RNA-dependent RNA polymerase needed for viral mRNA synthesis and genome replication. Producing all of these viral components within cultured cells was accomplished by cotransfecting plasmids for the VSV genomic cDNA plus expression plasmids that encode VSV N, P and L proteins.

All of these plasmids were designed for transcription by phage T7 RNA polymerase; accordingly transfected cells were also infected with a recombinant vaccinia virus that expresses the phage polymerase (MVA/T7 or VTF7-3). The standard procedure used for VSV rescue is described in Lawson *et al*. **1995**, cited above; and Schnell et al., 1996 J. Virol., 70:2318-23. This procedure was modified to make it more efficient for rescue of highly attenuated viruses, and also to make the procedure consistent with regulatory agency guidelines. Briefly, the method is described below.

Qualified Vero cells in 12.5 cm² flasks were transfected with a rVSV genomic clone and supported plasmids encoding the VSV *N, P* and *L* genes using a calcium-phosphate transfection procedure. At the time transfection was initiated, enough certified MVA/T7 was added to provide a multiplicity of infection of 2 plaque-forming units per cell. Three hours after the start of transfection, the cells were subjected to a 3 hour heat shock step to improve rescue efficiency of several negative strand RNA viruses (Parks, C. L. et al, 1999 J. Virol., 73:3560-6). At 48-72 hours after transfection, the cells and culture medium were transferred to a larger flask containing an established monolayer of Vero cells and subsequently incubated one or more days to allow amplification of rescued virus. Virus harvested after this amplification step was filtered to remove most contaminating MVA/T7, and used for clonal isolation of a rVSV strain.

The nucleotide sequence of the viral genomic RNA was determined by a consensus sequencing method to analyze RNA viral genomes. Briefly, purified viral RNA was reverse transcribed to produce cDNA, and then overlapping regions of the genome were amplified by PCR using gene-specific primers. PCR products were gel purified, then subjected to cycle-sequencing using fluorescent dye-terminators. Sequencing reaction products were purified and analyzed on an automated sequencer.

### C. Specific Constructs Used in the Examples

To produce the specific recombinant VSV vectors used in the following experiments, recombinant VSVs expressing the HIV-1 89.6P gp160 fused to the transmembrane region of the VSV G protein (rVSV HIV-lenvG) and SIVmac239 gag p55 (rVSV SIVgag) were mixed and used as the experimental immunogenic composition. A rVSV expressing the influenza hemagglutinin protein (rVSV fluHA) was used as a control composition. Recombinant VSV vectors were prepared as previously described (Rose et al, 2000 J. Virol. 74:10903-10) with the gene encoding the desired antigen inserted between the VSV G and L transcription units. The following construction was described in Rose *et al, supra.*

### 1. Plasmid construction.

A plasmid containing the Chandipura glycoprotein [G(Ch)] gene (Masters, P. S., 1989 Virol., 171:285-290) was kindly provided by Dr. Amiya Banerjee, Cleveland Clinic. To construct the VSV vector containing the *G*(Ch) gene in place of the Indiana glycoprotein [G(I)] gene, an *Xho*I site was first removed from within the *G*(Ch) gene using oligonucleotide-directed mutagenesis with the complementary primers 5'-CCCCTAGTGGGATCTCCAGTGATATTTGGAC (SEQ ID NO: 2) and 5'-GTCCAAATATCACTGGAGATCCCACTAGGGG (SEQ ID NO: 3) and the Stratagene QuikChange mutagenesis kit. The mutation of CTCGAG (SEQ ID NO: 4) to CTCCAG (SEQ ID NO: 5) eliminated the *Xho*I site without affecting the amino acid sequence of the G(Ch) protein. The gene was then amplified by PCR using Vent DNA polymerase (New England Biolabs). The forward primer was 5'-GATCGATCGAATTCACGCGTAACATGACTTCTTCAG (SEQ ID NO: 6), containing an *Mlu*I site (underlined) upstream of the ATG initiation codon for the G(Ch) protein. The reverse primer was 5'-GAACG**GTCGAC**GCGCC **TCGAG**CGTGATATCTGTTAGTTTTTTTCATATCATGTTGTTGGGCTTG AAGATC (SEQ ID NO: 7) and contained *Sal*I and *Xho*I sites (bold), followed by VSV transcription start and stop signals (underlined), followed by the complement of the 3' coding sequence of G(Ch).

The PCR product was digested with *Mlu*I and *Sal*I and cloned into the pVSVXN-1 vector (Schnell, et al 1996 J. Virol. 70:2318-2323) that had been digested with *Mlu*I and *Xho*I to remove the VSV G(I) coding sequence (*Sal*I and *Xho*I leave compatible ends for ligation). The plasmid derived by this method was designated pVSV(GCh)XN-1 and contains an expression site for foreign genes flanked by unique *Xho*I and *Nhe*I sites between the G(Ch) gene and the *L* gene.

A procedure identical to that described above was used to generate the vector containing the G(NJ) protein gene from plasmid pNJG (Gallione, C. J., and J. K. Rose. 1983 J. Virol. 46:162-169). The forward primer was 5'-GATCGATCGAA TTC**ACGCGT**AATATGTTGTCTTATCTAATCTTTGC (SEQ ID NO: 8), and the reverse primer was 5'-GGAACG**GTCGA**CGCGC**CTCGAG**CGTGATATCTGTT AGTTTTTTTCATATTAACGGAAATGAGCCATTTCCACG (SEQ ID NO: 9). The sites indicated by bold letters and the underlined sequences are as described for the Chandipura construction above, and the subsequent cloning steps were also as described above. The final vector plasmid derived was designated pVSV(GNJ)XN-1 and contains an expression site for foreign genes flanked by unique *Xho*I and *Nhe*I sites between the G(NJ) gene and the VSV *L* gene.

To generate the vectors containing the HIV *Env 89.6 G* gene, the gene encoding the 89.6 envelope protein with the VSV G cytoplasmic tail was excised with *Xho*I and *Nhe*I from pVSV-89.6gp160G (Johnson *et al* **1997**, cited above) and cloned between the *Xho*I and *Nhe*I sites in vector pVSV(GNJ)XN-1 or pVSV(GCh)XN-1. This *gp160G* gene encodes all of gp120 and the ecto- and transmembrane domains of gp41 and has four amino acids of the 89.6 Env cytoplasmic domain (N-R-V-R) (SEQ ID NO: 10) fused to the 26 C-terminal amino acids of the VSV G cytoplasmic domain, beginning with the sequence I-H-L-C (SEQ ID NO: 11).

### 2. Recoveries of recombinant viruses.

Recombinant VSVs were recovered using established methods (Lawson *et al* **1995** cited above). Briefly, baby hamster kidney (BHK) cells were grown to approximately 60% confluency on 10-cm dishes. The cells were then infected at a multiplicity of infection (MOI) of 10 with vTF7-3, a recombinant vaccinia virus that expresses T7 RNA polymerase (Fuerst et al 1987 Mol. Cell. Biol. 7:2538-2544). After 1 hour, each dish of cells was transfected with 3 µg of pBS-N, 5 µg of pBS-P, 1 µg of pBS-L, and 10 µg of the plasmid encoding one of the three full-length recombinants described above. Transfections were performed with a cationic liposome reagent containing dimethyldioctadecyl ammonium bromide and dioleyl-phosphatidylethanolamine (Rose et al, 1991 Biotechniques 10: 520-525). Cells were then incubated at 37°C for 48 hours. Cell supernatants were passed through a 0.2-µm filter to remove the majority of the vaccinia virus and then applied to fresh BHK cells for an additional 48 hours at 37°C. For some recoveries, an additional plasmid encoding VSV G (pBS-G), 4 µg/plate, was included with the N, P, and L support plasmids. Recovery of infectious virus was confirmed by scanning BHK cell monolayers for VSV cytopathic effect. Virus plaques were then isolated on BHK cells, and virus stocks from individual plaques were grown by adding virus from a single plaque to a 10-cm-diameter plate of BHK cells. These stocks were then stored at 80°C. The titers obtained for VSV(GI)-89.6G, VSV(GCh)-89.6G, and VSV(GNJ)-89.6G were all in the range of 10⁷ to 10⁸ PFU/ml after freezing and thawing, a procedure that reduces VSV titers approximately threefold.

For use in the following experiments, each rVSV construct was diluted to a final volume of 0.8cc with sterile DME.

### EXAMPLE 3: PRIME/BOOST IMMUNIZATION REGIMEN

### A. Immunization Protocols

Rhesus macaques (5 per group) were immunized by intramuscular injection with 5 mgs of a bicistronic DNA plasmid encoding rhesus IL-12 p35 and IL-12 p40 in combination with 5 mgs of a DNA plasmid expressing SIV gag p37 polyprotein (Groups 1 and 2), or 10 mgs of an empty DNA plasmids (Groups 3 and 4). All these DNA plasmids and the formulations thereof are described in detail in Example 1. The DNA immunization schedule provided for an initial immunization at day 0, followed by a first and second booster immunization at week 4 and week 8. The injections were made at four sites in the deltoids and quadriceps with 1cc per site using a needle and syringe.

The macaques were then boosted at week 15 by intranasal inoculation (0.4 cc/nostril with handheld pipetter) with either the recombinant vesicular stomatitus virus (rVSV) of serotype Indiana (I) based vector of Example 2 containing HIV-1 *gp160 env* gene (5 x 10⁶ pfu) and a second rVSV(I) containing SIV *gag p55* gene (5 x 10⁶ pfu) (Groups 2 and 3), or rVSV(I) containing influenza hemagglutinin gene (1x 10⁷ pfu) (Groups 1 and 4). The macaques were again boosted at week 23 by intranasal inoculation (0.4 cc/nostril with handheld pipetter) with either the rVSV (serotype Chandipuri, Ch) based vector of Example 2 containing HIV-1 *gp160 env* gene (5 x 10⁶ pfu) and a second rVSV(Ch) containing SIV*gag p55* gene (5 x 10⁶ pfu) (Groups 2 and 3), or rVSV(Ch) containing influenza hemagglutinin gene (1x 10⁷ pfu) (Groups 1 and 4).

Macaques were closely monitored for the induction of cellular and humoral immune responses in peripheral blood and for antibody responses at various mucosal surfaces. This monitoring involved the performance of the enzyme-linked immunospot assay (ELISpot) for SIV gag p55 peptide pool for gamma interferon, an ELISpot for HIV-1 env 6101 peptide pool for gamma interferon and an ELISpot for VSV N peptide pool for gamma interferon. The ELISpot assay detected cellular immune responses in the PBL.

Humoral immune responses are examined by evaluating the serum (FIG. 4), nasal wash, rectal secretions and saliva (data not shown) for anti-SIV gag p27 IgG titers by ELISA and anti-HIV-1gp160 env titers by ELISA (data not shown). For the serum, the antibodies employed in the ELISA were normal antibodies to the chimeric viruses SHIV89.6 and SHIV89.

### B. ELISpot Assay to detect cytokine-secreting murine and human cells

The filter immunoplaque assay, otherwise called the enzyme-linked immunospot assay (ELISpot), was initially developed to detect and quantitate individual antibody-secreting B cells. The technique originally provided a rapid and versatile alternative to conventional plaque-forming cell assays. Recent modifications have improved the sensitivity of the ELISpot assay such that cells producing as few as 100 molecules of specific protein per second can be detected. These assays take advantage of the relatively high concentration of a given protein (such as a cytokine) in the environment immediately surrounding the protein-secreting cell. These cell products are captured and detected using high-affinity antibodies.

The ELISpot assay utilizes two high-affinity cytokine-specific antibodies directed against different epitopes on the same cytokine molecule: either two monoclonal antibodies or a combination of one monoclonal antibody and one polyvalent antiserum. ELISpot generates spots based on a colorimetric reaction that detects the cytokine secreted by a single cell. The spot represents a "footprint" of the original cytokine-producing cell. Spots (i.e., spot forming cells or SFC) are permanent and can be quantitated visually, microscopically, or electronically.

The ELISpot assay was performed as follows: Ninety-six-well flat-bottom ELISpot plates (Millipore, Bedford, MA) were coated overnight with a mouse antihuman γ interferon (hIFN-γ) monoclonal antibody (clone 27, BD-Pharmingen, San Diego CA) at a concentration of 1 µg/mL, washed ten times with 1x PBS supplemented with 0.25% Tween-20, and blocked for 2 hours with PBS containing 5% fetal bovine serum (FBS). Rhesus macaque peripheral blood lymphocytes (PBLs) were isolated from freshly drawn heparinized whole blood by Ficoll-Hypaque density gradient centrifugation and resuspended in complete culture medium (RPMI 1640 medium supplemented with 5% FCS, 2 mM L-glutamine, 100 units/mL penicillin, 100 µg/mL streptomycin sulfate, 1 mM sodium pyruvate, 1 mM HEPES, 100 µM non-essential amino acids) containing either 50 µg/mL PHA-M (Sigma), a pool of 15 amino acid peptides over lapping by 11 amino acids spanning the entire SIV gag open reading frame (1µM final peptide concentration), or medium alone.

Input cell numbers were 2 x 10⁵ PBLs in 100 µL/well and assayed in duplicate wells. Cells were incubated for 16 hours at 37°C and then removed from the plate by washing first with deionized water and then 10 times with 1x PBS containing 0.25% Tween-20. Thereafter, the plates were treated with a rabbit polyclonal anti-hIFN-γ biotinylated detector antibody (0.2 µg/well, Biosource, Camarillo, CA) diluted with 1x PBS containing 1% BSA and incubated at room temperature for 2 hours. Plates were then washed 10 times with 1x PBS containing 0.25% Tween-20 and treated with 100 µL per well of streptavidin-alkaline phosphatase conjugate (Southern Biotech, Birmingham AL) diluted 1:500 with 1x PBS containing 5% FBS and 0.005% Tween-20 and incubated an additional 2.5 hours at room temperature. Unbound conjugate was removed by rinsing the plate 10 times with 1x PBS containing 0.25% Tween-20. Chromogenic substrate (100 µL/well, 1-step NBT/BCIP, Pierce, Rockford, IL) was then added for 3-5 minutes, rinsed away with water, the plate air-dried, and resulting spots were counted by eye using an inverted dissecting microscope.

The performance of the ELISpot assay to the present invention measured the number of CD8+ T cells (CTLs) and CD4+ T cells induced in response to the prime/boost immunization method of this invention, as measured by the production of gamma interferon. This assessment was tracked through week 25 for the above-treated macaques (after 3 DNA primes and 2 VSV boosts).

### C. Results

After primary DNA immunization, SIVgag-specific IFN-γ ELISpot responses were readily detected in 8 of 10 SIV gag/rIL-12 DNA immunized macaques (mean 254 SFC/10⁶ cells). After the second SIV gag/IL-12 DNA immunization, 10 of 10 immunized macaques developed high level ELISpot responses (mean 1133 SFC/10⁶ cells) and a third dose of gag/IL-12 DNA boosted the gag-specific ELISpot responses in a majority of animals (mean 1506 SFC/10⁶ cells). After the first rVSV HIVenv/rVSV SIVgag boost, mean SIV gag ELISpot responses were substantially higher (3772 SFC/10⁶ cells) than responses receiving only a single rVSV HIVenv/rVSV SIVgag immunization (mean 386 SFC/10⁶ cells). These results support the use of a cytokine enhanced DNA prime to augment the immunogenicity of rVSV immunization.

These results are reported in Table 1 below and in FIGs. 2, 3, 4 and 5. FIG. 2 shows the rVSV N-specific IFN-γ ELISpot responses in unfractionated peripheral blood mononuclear cells (PBMC) from these animals immunized after week 25. FIG. 3 shows the HIVenv 6101-specific IFN-γELISpot responses for the same samples. Asterisks indicate where statistically significant differences occurred at p=0-.0001. FIG. 4 shows the results of the serum antibody responses measuring anti-SIV gag p27 IgG titers by ELISA and anti-HIV-lgp160 env titers by ELISA. A protocol of immunization with the DNA plasmid encoding the SIV gag protein with a boost of a VSV vector expressing flu HA protein is represented by (◆). A protocol of the invention involving a priming DNA gag plasmid immunization followed by a VSV boost expressing the HIV gag and env proteins is represented by (■). A protocol involving a priming immunization with an empty control DNA followed by immunization with a VSV expressing the HIV gag and env proteins is represented by (▲). A protocol involving a priming immunization with control DNA plasmid followed by immunization with a VSV expressing flu HA protein is represented by (●). Each group represents results from 5 animals. Statistically significant differences between groups are shown as p=0.0073 (*); p=0.5941 (#) or p=0.0027 (¥).

FIG. 5 shows the mean SIV gag-specific IFN-γ ELISpot responses for the same samples. A protocol of immunization with the DNA plasmid encoding the SIV gag protein with a boost of a VSV vector expressing flu HA protein is represented by (◆). A protocol of the invention involving a priming DNA gag plasmid immunization followed by a VSV boost expressing the HIV gag and env proteins is represented by (■). A protocol involving a priming immunization with an empty control DNA followed by immunization with a VSV expressing the HIV gag and env proteins is represented by (▲). The (●) represent a protocol involving a priming immunization with control DNA plasmid followed by immunization with a VSV expressing flu HA protein. Each group represents results from 5 animals. Statistically significant differences between groups are indicated by brackets for p=0.0001 and p=0.0002.

Table 1 reports the same results in tabular form.

**Table 1: Mean SIV gag-specific IFN-gamma ELISpot responses in rhesus macaques following a SIV gag/rhesus IL-12 DNA prime and a rVSV-SIVgag/rVSV-HIVenv boost**

| Group #/Protocol/ No. of Animals | Prime | Boost | Mean SIVgag-specific IFNγ ELISpot response (#SFC/10⁶ PBLs)^{a} |
|---|---|---|---|
| 1, prime only (n=5) | SIVgag/IL-12 DNA | VSV FluHA | 471 |
| 2, prime/boost (n=5) | SIVgag/IL-12 DNA | VSV-SIVgag/HIVenv | 2,338 |
| 3, boost only (n=5) | empty DNA | VSV-SIVgag/HIVenv | 420 |
| 4, control (n=5) | empty DNA | VSV FluHA | 55 |

| | | | |
|---|---|---|---|
| ^{a} mean SIV gag-specific ELISpot responses are reported at week 25 after the initial DNA immunization. | | | |

The results of these assays demonstrate a surprising synergistic effect of a prime/boost regimen according to this invention, when compared to the results of administering multiple priming compositions only and multiple boosting compositions only. The combination of the presentation of the desired antigen by a DNA plasmid administration followed by a rVSV boost produces an increase in antigen-specific T cells in the immunized subject, that is considerably in excess of any additive response. Similarly, the increase demonstrated in the humoral response to the desired antigen is unexpectedly high with the use of both the DNA plasmid and rVSV immunogenic compositions in an immunization protocol.

### EXAMPLE 4: MACAQUE MODEL FOR IMMUNIZATION AGAINST HIV

Rhesus (Rh) macaques are immunized using the prime/boost strategy of Example 3 and the plasmids and VSV vectors described therein according to the same protocols. About 32 weeks after the first priming composition administration, the macaques are challenged with a dose of 330 50% monkey infectious doses (MID₅₀) of pathogenic SIV/HIV recombinant virus SHIV89.6P (Reimann et al, 1996 J. Virol., 70:3198-3206; Reimann et al 1996 J. Virol., 70:6922-6928).

About 50-70 days following challenge, the animals are monitored for disease. Animals are monitored for the induction of cell-mediated and antibody responses immediately prior to, and one and two weeks after each immunization. Serum collected prior to challenge and 2, 4, 6, 8 and 12 weeks after challenge is tested for neutralizing antibody responses against HIV-1 89.6, 89.6P, 6101 and other Clade B primary isolates. During the immunization phase of the experiment, immediately prior to and every two weeks after challenge, CD4/CD8 counts are monitored. Immediately before and every week after challenge, viral loads in serum are determined by branch DNA analysis. Other body fluids (vaginal, rectal, and nasal secretions, as well as saliva) of the animals are examined for cellular and humoral immune responses.

Cell-mediated immune responses to the desired antigen are analyzed using several of the most appropriate cell based assays, which include the ⁵¹Cr-release CTL assay, soluble MHC Class I tetramer staining, ELISpot assay, and intracellular cytokine analysis. Mucosal antibody responses are evaluated by ELISA techniques optimized for use with mucosal samples. Serum antibody responses are evaluated by standard ELISA techniques. In addition, serum from all immunized animals is examined for neutralization antibody responses against HIV Env 6101 and other primary Clade B isolates. FIG. 6 illustrates that the elevated immune responses elicited by the prime/boost combinations of this invention result in increased protection from AIDS, as measured by a decreased loss of CD4 T-cells cells for at least 250 days post-challenge.

In addition to monitoring immune responses elicited by the immunogenic composition, the live virus vector's transmission potential is assessed by determining the level and duration for which it is shed. Nasal washes obtained at frequent intervals during the first three weeks after each immunization are tested for the presence of live VSV. Plasma viral load is also examined for the presence of live virus copies. FIG. 7 demonstrates that the elevated immune responses elicited by the prime/boost combinations result in a decrease in circulating virus in plasma for at least 250 days post-challenge.

The results of such examination are also likely to be very high antigen-specific CD8+ and CD4+ T cells in the animals immunized according to this invention in contrast with control animals. It is anticipated that animal immunized according to the prime/boost methodology of this invention will remain healthy after HIV exposure, while unimmunized animals will develop AIDS.

A comparison of the results of this protocol with other known prime/boost methodologies is anticipated to demonstrate that the method of the present invention has advantages in safety for the immunized animals and in eliciting higher levels of anti-HIV CTLs and antibodies than provided by immunization with the one or multiple DNA priming composition immunizations alone or with one or multiple rVSV vector immunizations alone. Repeated prime/boost according to this invention is likely to be synergistic and thus both prophylactically beneficial to pre-exposed subjects and therapeutically beneficial to subjects already infected with HIV.

Various modifications and minor alterations in the method and components are believed to be clear to those of skill in the art.

## Claims

1. Use of a first composition comprising a DNA plasmid comprising a DNA sequence encoding an antigen under the control of regulatory sequences directing expression thereof by said DNA plasmid in the preparation of a medicament for inducing an antigen specific immune response in a mammalian subject, wherein inducing said antigen specific immune response comprises
(a) administering to said subject an effective amount of said first composition; and
(b) administering to said subject an effective amount of a second composition comprising a recombinant vesicular stomatitis virus (VSV) comprising a nucleic acid sequence encoding said antigen under the control of regulatory sequences directing expression thereof by said recombinant VSV.

2. Use of a second composition comprising a recombinant vesicular stomatitis virus (VSV) comprising a nucleic acid sequence encoding an antigen under the control of regulatory sequences directing expression thereof by said recombinant VSV in the preparation of a medicament for inducing an antigen specific immune response in a mammalian subject, wherein inducing said antigen specific immune response comprises
(a) administering to said subject an effective amount of a first composition comprising a DNA plasmid comprising a DNA sequence encoding said antigen under the control of regulatory sequences directing expression thereof by said DNA plasmid; and
(b) administering to said subject an effective amount of said second composition.

3. Use of
(a) a first composition comprising a DNA plasmid comprising a DNA sequence encoding an antigen under the control of regulatory sequences directing expression thereof by said DNA plasmid; and
(b) a second composition comprising a recombinant vesicular stomatitis virus (VSV) comprising a nucleic acid sequence encoding said antigen under the control of regulatory sequences directing expression thereof by said recombinant VSV, in the preparation of a medicament for inducing an antigen specific immune response in a mammalian subject.

4. Use according to any one of claims 1 to 3, wherein said VSV is replication competent or non-replicating.

5. Use according to any one of claims 1 to 3, wherein said first composition is administered to said subject at least once prior to administration of said second composition.

6. Use according to claim 5, wherein said second composition is administered to said subject at least once.

7. Use according to any one of claims 1 to 3, wherein said second composition is administered to said subject at least once prior to administration of said first composition.

8. Use according to claim 7, wherein said first composition is administered to said subject at least once.

9. Use according to any one of claims 1 to 3, wherein said medicament further comprises a cytokine, and wherein (a) or (b) further comprises administering an effective amount of said cytokine to said mammal.

10. Use according to claim 9, wherein said cytokine administered in (a) is a nucleic acid composition comprising a DNA plasmid comprising a DNA sequence encoding a cytokine under the control of regulatory sequences directing expression thereof by said DNA plasmid.

11. Use according to claim 9, wherein said cytokine administered in (b) is a protein.

12. Use according to claim 10 or claim 11, wherein said cytokine is selected from the group consisting of IL-12, IL-15, GM-CSF, and a combination thereof.

13. Use according to claim 10, wherein said cytokine-encoding sequence is present on the same DNA plasmid as said antigen-encoding sequence or is present on a DNA plasmid different from said DNA plasmid encoding said antigen.

14. Use according to any one of claims 1 to 3, wherein said antigen is a protein, polypeptide, peptide, a fragment or a fusion thereof, wherein said protein is derived from a member selected from the group consisting of a bacterium, virus, fungus, parasite, a cancer cell, an allergen and a self-molecule.

15. Use according to claim 14, wherein said antigen is a human or simian immunodeficiency virus antigen selected from the group consisting of *gag, pol, env, nef, vpr, vpu, vif* and *tat,* and immunogenic fragments or fusions thereof.

16. Use according to any one of claims 1 to 3, wherein said first composition comprises (i) one DNA plasmid comprising a DNA sequence encoding more than one copy of the same or a different said antigen or (ii) more than one DNA plasmid, wherein each DNA plasmid encodes the same or a different antigen.

17. Use according to any one of claims 1 to 3, wherein said medicament further comprises at least two said recombinant VSVs, and wherein (b) further comprises administering at least two said recombinant VSVs.

18. Use according to claim 17, wherein each said recombinant VSV has a (i) different VSV G protein and different VSV serotype, but the same antigen encoding sequence, (ii) different antigen encoding sequence, but the same VSV G protein, or (iii) different antigen encoding sequence and a different VSV G protein.

19. Use according to any one of claims 1 to 3, wherein said second composition comprises at least two said rVSVs, wherein each rVSV carried a G gene from a different VSV serotype.

20. Use according to any one of claims 1 to 3, wherein administering said second composition comprises serially administering at least two rVSV composition.

21. A kit for inducing an antigen specific immune response in a mammalian subject comprising
at least one first composition comprising a DNA plasmid comprising a DNA sequence encoding an antigen under the control of regulatory sequences directing expression thereof by said DNA plasmid; and
at least one second composition comprising a recombinant vesicular stomatitis virus (VSV) comprising a nucleic acid sequence encoding said antigen under the control of regulatory sequences directing expression thereof by said recombinant VSV.

22. The kit according to claim 21, wherein said VSV is replication competent or non-replicating.

23. The kit according to claim 21, further comprising a cytokine composition.

24. The kit according to claim 23, wherein said cytokine composition comprises a nucleic acid composition comprising a DNA plasmid comprising a DNA sequence encoding said cytokine under the control of regulatory sequences directing expression thereof by said DNA plasmid.

## Patentansprüche

1. Verwendung einer ersten Zusammensetzung, die ein DNA-Plasmid umfasst, das eine DNA-Sequenz umfasst, die ein Antigen unter der Kontrolle von Regulationssequenzen codiert, die dessen Expression durch das DNA-Plasmid steuern, bei der Herstellung eines Arzneimittels zur Induzierung einer antigenspezifischen Immunantwort in einem Säugetierprobanden, wobei die Induzierung der antigenspezifischen Immunantwort Folgendes umfasst:
(a) Verabreichen einer wirksamen Menge der ersten Zusammensetzung an den Probanden und
(b) Verabreichen einer wirksamen Menge einer zweiten Zusammensetzung an den Probanden, die ein rekombinantes Virus der vesikulären Stomatitis (VSV) umfasst, das eine Nukleinsäuresequenz umfasst, die das Antigen unter der Kontrolle von Regulationssequenzen codiert, die dessen Expression durch das rekombinante Virus steuern.

2. Verwendung einer zweiten Zusammensetzung, die ein rekombinantes Virus der vesikulären Stomatitis (VSV) umfasst, das eine Nukleinsäuresequenz umfasst, die das Antigen unter der Kontrolle von Regulationssequenzen codiert, die dessen Expression durch das rekombinante VSV steuern, bei der Herstellung eines Arzneimittels zur Induzierung einer antigenspezifischen Immunantwort in einem Säugetierprobanden, wobei die Induzierung der antigenspezifischen Immunantwort Folgendes umfasst:
(a) Verabreichen einer wirksamen Menge einer ersten Zusammensetzung an den Probanden, die ein DNA-Plasmid umfasst, das eine DNA-Sequenz umfasst, die ein Antigen unter der Kontrolle von Regulationssequenzen codiert, die dessen Expression durch das DNA-Plasmid steuern; und
(b) Verabreichen einer wirksamen Menge der zweiten Zusammensetzung an den Probanden.

3. Verwendung von
(a) einer ersten Zusammensetzung, die ein DNA-Plasmid umfasst, das eine DNA-Sequenz umfasst, die ein Antigen unter der Kontrolle von Regulationssequenzen codiert, die dessen Expression durch das DNA-Plasmid steuern; und
(b) einer zweiten Zusammensetzung, die ein rekombinantes Virus der vesikulären Stomatitis (VSV) umfasst, das eine Nukleinsäuresequenz umfasst, die das Antigen unter der Kontrolle von Regulationssequenzen codiert, die dessen Expression durch das rekombinante VSV steuern, bei der Herstellung eines Arzneimittels zur Induzierung einer antigenspezifischen Immunantwort in einem Säugetierprobanden.

4. Verwendung nach einem der Ansprüche 1 bis 3, wobei das VSV replikationsfähig oder nicht replizierend ist.

5. Verwendung nach einem der Ansprüche 1 bis 3, wobei die erste Zusammensetzung mindestens einmal vor der Verabreichung der zweiten Zusammensetzung an den Probanden verabreicht wird.

6. Verwendung nach Anspruch 5, wobei die zweite Zusammensetzung mindestens einmal an den Probanden verabreicht wird.

7. Verwendung nach einem der Ansprüche 1 bis 3, wobei die zweite Zusammensetzung mindestens einmal vor der Verabreichung der ersten Zusammensetzung an den Probanden verabreicht wird.

8. Verwendung nach Anspruch 7, wobei die erste Zusammensetzung mindestens einmal an den Probanden verabreicht wird.

9. Verwendung nach einem der Ansprüche 1 bis 3, wobei das Arzneimittel weiterhin ein Cytokin umfasst und wobei (a) oder (b) weiterhin das Verabreichen einer wirksamen Menge des Cytokins an das Säugetier umfasst.

10. Verwendung nach Anspruch 9, wobei es sich bei dem Cytokin, das in (a) verabreicht wird, um eine Nukleinsäurenzusammensetzung handelt, die ein DNA-Plasmid umfasst, das eine DNA-Sequenz umfasst, die ein Cytokin unter der Kontrolle von Regulationssequenzen codiert, die dessen Expression durch das DNA-Plasmid steuern.

11. Verwendung nach Anspruch 9, wobei es sich bei dem Cytokin, das in (b) verabreicht wird, um ein Protein handelt.

12. Verwendung nach Anspruch 10 oder 11, wobei das Cytokin aus der Gruppe bestehend aus IL-12, IL-15, GM-CSF und eine Kombination davon ausgewählt ist.

13. Verwendung nach Anspruch 10, wobei die cytokincodierende Sequenz auf demselben DNA-Plasmid wie die antigencodierende Sequenz vorliegt oder auf einem DNA-Plasmid vorliegt, das sich von dem DNA-Plasmid unterscheidet, das das Antigen codiert.

14. Verwendung nach einem der Ansprüche 1 bis 3, wobei es sich bei dem Antigen um ein Protein, ein Polypeptid, ein Peptid, ein Fragment oder eine Fusion davon handelt, wobei das Protein von einem Element abgeleitet ist, das aus der Gruppe bestehend aus einem Bakterium, einem Virus, einem Pilz, einem Parasiten, einer Krebszelle, einem Allergen und einem Selbstmolekül ausgewählt ist.

15. Verwendung nach Anspruch 14, wobei es sich bei dem Antigen um ein Immundefektvirus-Antigen eines Menschen oder eines Affen handelt, das aus der Gruppe bestehend aus *gag, pol, env, nef, vpr, vpu, vif* und *tat* und immunogenen Fragmenten und Fusionen davon ausgewählt ist.

16. Verwendung nach einem der Ansprüche 1 bis 3, wobei die erste Zusammensetzung (i) ein DNA-Plasmid, die eine DNA-Sequenz umfasst, die mehr als eine Kopie desselben oder eines anderen Antigens codiert, oder (ii) mehr als ein DNA-Plasmid umfasst, wobei jedes DNA-Plasmid dasselbe oder jeweils ein anderes Antigen codiert.

17. Verwendung nach einem der Ansprüche 1 bis 3, wobei das Arzneimittel weiterhin mindestens zwei rekombinante VSV umfasst und wobei (b) weiterhin das Verabreichen von mindestens zwei der rekombinanten VSV umfasst.

18. Verwendung nach Anspruch 17, wobei jedes rekombinante VSV ein (i) jeweils anderes VSV-G-Protein und einen jeweils anderen VSV-Serotyp, jedoch die gleiche antigencodierende Sequenz, (ii) eine jeweils andere antigencodierende Sequenz, jedoch das gleiche VSV-G-Protein oder (iii) eine jeweils andere antigencodierende Sequenz und ein jeweils anderes VSV-G-Protein aufweist.

19. Verwendung nach einem der Ansprüche 1 bis 3, wobei die zweite Zusammensetzung mindestens zwei der rVSV umfasst, wobei jedes rVSV ein G-Gen von einem jeweils anderen VSV-Serotyp trägt.

20. Verwendung nach einem der Ansprüche 1 bis 3, wobei das Verabreichen der zweiten Zusammensetzung das serielle Verabreichen von mindestens zwei rVSV-Zusammensetzungen umfasst.

21. Kit zum Induzieren einer antigenspezifischen Immunantwort in einem Säugetierprobanden, das Folgendes umfasst:
mindestens eine erste Zusammensetzung, die ein DNA-Plasmid umfasst, das eine DNA-Sequenz umfasst, die ein Antigen unter der Kontrolle von Regulationssequenzen codiert, die dessen Expression durch das DNA-Plasmid steuern; und
mindestens eine zweite Zusammensetzung, die ein rekombinantes Virus der vesikulären Stomatitis (VSV) umfasst, das eine Nukleinsäuresequenz umfasst, die das Antigen unter der Kontrolle von Regulationssequenzen codiert, die dessen Expression durch das rekombinante VSV steuern.

22. Kit nach Anspruch 21, wobei das VSV replikationsfähig oder nicht replizierend ist.

23. Kit nach Anspruch 21, das weiterhin eine Cytokinzusammensetzung umfasst.

24. Kit nach Anspruch 23, wobei die Cytokinzusammensetzung eine Nukleinsäurenzusammensetzung umfasst, die ein DNA-Plasmid umfasst, das eine DNA-Sequenz umfasst, die ein Cytokin unter der Kontrolle von Regulationssequenzen codiert, die dessen Expression durch das DNA-Plasmid steuern.

## Revendications

1. Utilisation d'une première composition comprenant un plasmide ADN comprenant une séquence d'ADN codant un antigène sous le contrôle de séquences de régulation dirigeant l'expression de celui-ci par ledit plasmide ADN dans la préparation d'un médicament pour induire une réponse immune spécifique d'un antigène chez un sujet du type mammifère, dans lequel l'induction de ladite réponse immune spécifique d'un antigène comprend
(a) l'administration audit sujet d'une quantité efficace de ladite première composition ; et
(b) l'administration audit sujet d'une quantité efficace d'une seconde composition comprenant un virus recombinant de la stomatite vésiculaire (VSV) comprenant une séquence d'acide nucléique codant ledit antigène sous le contrôle de séquences de régulation dirigeant l'expression de celui-ci par ledit VSV recombinant.

2. Utilisation d'une seconde composition comprenant un virus recombinant de la stomatite vésiculaire (VSV) comprenant une séquence d'acide nucléique codant un antigène sous le contrôle de séquences de régulation dirigeant l'expression de celui-ci par ledit VSV recombinant dans la préparation d'un médicament pour l'induction d'une réponse immune spécifique d'un antigène chez un sujet du type mammifère, dans laquelle l'induction de ladite réponse immune spécifique d'un antigène comprend
(a) l'administration audit sujet d'une quantité efficace d'une première composition comprenant un plasmide ADN comprenant une séquence d'ADN codant ledit antigène sous le contrôle de séquences de régulation dirigeant l'expression de celui-ci par ledit plasmide ADN ; et
(b) l'administration audit sujet d'une quantité efficace de ladite seconde composition.

3. Utilisation de
(a) une première composition comprenant un plasmide ADN comprenant une séquence d'ADN codant un antigène sous le contrôle de séquences de régulation dirigeant l'expression de celui-ci par ledit plasmide ADN ; et
(b) une seconde composition comprenant un virus recombinant de la stomatite vésiculaire (VSV) comprenant une séquence d'acide nucléique codant ledit antigène sous le contrôle de séquences de régulation dirigeant l'expression de celui-ci par ledit VSV recombinant, dans la préparation d'un médicament pour l'induction d'une réponse immune spécifique d'un antigène chez un sujet du type mammifère.

4. Utilisation selon l'une quelconque des revendications 1 à 3, dans laquelle ledit VSV est apte à se répliquer ou inapte à se répliquer.

5. Utilisation selon l'une quelconque des revendications 1 à 3, dans laquelle ladite première composition est administrée audit sujet au moins une fois avant l'administration de ladite seconde composition.

6. Utilisation selon la revendication 5, dans laquelle ladite seconde composition est administrée audit sujet au moins une fois.

7. Utilisation selon l'une quelconque des revendications 1 à 3, dans laquelle ladite seconde composition est administrée audit sujet au moins une fois avant l'administration de ladite première composition.

8. Utilisation selon la revendication 7, dans laquelle ladite première composition est administrée audit sujet au moins une fois.

9. Utilisation selon l'une quelconque des revendications 1 à 3, dans laquelle ledit médicament comprend en outre une cytokine, et dans laquelle (a) ou (b) comprend en outre l'administration d'une quantité efficace de ladite cytokine audit mammifère.

10. Utilisation selon la revendication 9, dans laquelle ladite cytokine administrée en (a) est une composition d'acide nucléique comprenant un plasmide ADN comprenant une séquence d'ADN codant une cytokine sous le contrôle de séquences de régulation dirigeant l'expression de celle-ci par ledit plasmide ADN.

11. Utilisation selon la revendication 9, dans laquelle ladite cytokine administrée en (b) est une protéine

12. Utilisation selon la revendication 10 ou la revendication 11, dans laquelle ladite cytokine est choisie parmi le groupe constitué de l'IL-12, IL-15, du GM-CSF, et d'une combinaison de ceux-ci.

13. Utilisation selon la revendication 10, dans laquelle ladite séquence codant une cytokine est présente sur le même plasmide ADN que ladite séquence codant l'antigène ou est présente sur un plasmide ADN différent dudit plasmide ADN codant ledit antigène.

14. Utilisation selon l'une quelconque des revendications 1 à 3, dans laquelle ledit antigène est une protéine, un polypeptide, un peptide, un fragment ou une fusion de ceux-ci, dans laquelle ladite protéine est dérivée d'un élément choisi parmi le groupe constitué d'une bactérie, d'un virus, d'un champignon, d'un parasite, d'une cellule cancéreuse, d'un allergène et d'une molécule du soi

15. Utilisation selon la revendication 14, dans laquelle ledit antigène est un antigène du virus de l'immunodéficience humaine ou simienne choisi parmi le groupe constitué de *gag, pol, env, nef, vpr, vpu, vif et tat,* et de fragments immunogéniques ou de fusions de ceux-ci.

16. Utilisation selon l'une quelconque des revendications 1 à 3, dans laquelle ladite première composition comprend (i) un plasmide ADN comprenant une séquence d'ADN codant plus d'une copie dudit même antigène ou antigène différent ou (ii) plus d'un plasmide ADN, dans laquelle chaque plasmide ADN code le même antigène ou un antigène différent.

17. Utilisation selon l'une quelconque des revendications 1 à 3, dans laquelle ledit médicament comprend en outre au moins deux desdits VSV recombinants, et dans laquelle (b) comprend en outre l'administration d'au moins deux desdits VSV recombinants.

18. Utilisation selon la revendication 17, dans laquelle chacun desdits VSV recombinants a une (i) protéine G de VSV différente et un différent sérotype de VSV, mais la même séquence codant l'antigène, (ii) une différente séquence codant l'antigène, mais la même protéine G de VSV, ou (iii) une différente séquence codant l'antigène et une différente protéine G de VSV.

19. Utilisation selon l'une quelconque des revendications 1 à 3, dans laquelle ladite seconde composition comprend au moins deux desdits rVSV, dans laquelle chaque rVSV porte un gène G provenant d'un sérotype de VSV différent.

20. Utilisation selon l'une quelconque des revendications 1 à 3, dans laquelle l'administration de ladite seconde composition comprend l'administration en série d'au moins deux compositions de rVSV.

21. Kit d'induction d'une réponse immune spécifique d'un antigène chez un sujet du type mammifère comprenant
au moins une première composition comprenant un plasmide ADN comprenant une séquence d'ADN codant un antigène sous le contrôle de séquences de régulation dirigeant l'expression de celui-ci par ledit plasmide ADN ; et
au moins une seconde composition comprenant un virus recombinant de la stomatite vésiculaire (VSV) comprenant une séquence d'acide nucléique codant ledit antigène sous le contrôle de séquences de régulation dirigeant l'expression de celui-ci par ledit VSV recombinant.

22. Kit selon la revendication 21, dans lequel ledit VSV est apte à la réplication ou inapte à la réplication.

23. Kit selon la revendication 21, comprenant en outre une composition de cytokine.

24. Kit selon la revendication 23, dans lequel ladite composition de cytokine comprend une composition d'acide nucléique comprenant un plasmide ADN comprenant une séquence d'ADN codant ladite cytokine sous le contrôle de séquences de régulation dirigeant l'expression de celle-ci par ledit plasmide ADN.
